(19) Europäisches Patentamt
European Patent Office
Office européen des brevets

(11) Veröffentlichungsnummer : **0 507 736 A1**

(12) **EUROPÄISCHE PATENTANMELDUNG**

(21) Anmeldenummer : **92810225.0**

(22) Anmeldetag : **26.03.92**

(51) Int. Cl.$^5$ : **C07D 213/40,** C07D 277/32, C07D 213/61, C07D 213/89, A01N 43/40, A01N 43/78, A01N 47/42

(30) Priorität : **04.04.91 CH 1004/91**

(43) Veröffentlichungstag der Anmeldung : **07.10.92 Patentblatt 92/41**

(84) Benannte Vertragsstaaten : **AT BE CH DE DK ES FR GB GR IT LI LU NL PT**

(71) Anmelder : **CIBA-GEIGY AG**
**Klybeckstrasse 141**
**CH-4002 Basel (CH)**

(72) Erfinder : **Kristiansen, Odd, Dr.**
**Delligrabenstrasse 7**
**CH-4313 Möhlin (CH)**
Erfinder : **Gsell, Laurenz, Dr.**
**Maiengasse 56**
**CH-4056 Basel (CH)**
Erfinder : **Maienfisch, Peter, Dr.**
**Aegertenstrasse 21**
**CH-4118 Rodersdorf (CH)**

(54) **Heterozyklische Amidinderivate und ihre Verwendung als Schädlingsbekämpfungsmittel.**

(57) Verbindungen der Formel

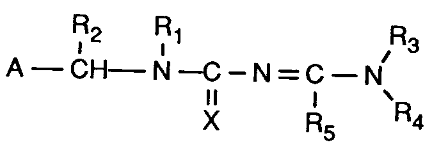

(I),

worin
$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl und
$R_4$ für $C_1$-$C_4$-Alkyl, $C_3$-$C_6$-Cycloalkyl oder $-CH_2CH_2COOR_7$ oder
$R_3$ und $R_4$ gemeinsam für $-(CH_2)_4-$ oder $-(CH_2)_5-$,
$R_5$ für Wasserstoff, $C_1$-$C_4$-Alkyl,

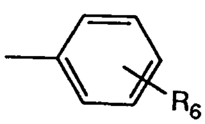

,

$-CH_2OCH_3$, -CN, $-COOR_7$ oder

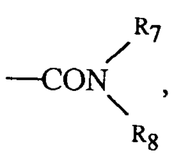

,

$R_6$ für Wasserstoff, Chlor, Methyl oder Nitro,
$R_7$ und $R_6$ für Methyl oder Ethyl,
A für einen unsubstituierten oder ein- bis vierfach substituierten, aromatischen oder nicht-aromatischen, monocyclischen oder bicyclischen, heterocyclischen Rest, wobei ein bis zwei Substituenten aus der Gruppe, bestehend aus $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyano und Nitro, und ein bis vier Substituenten aus der Gruppe, bestehend aus $C_1$-$C_3$-Alkyl, $C_1$-$C_3$-Alkoxy und Halogen, ausgewählt sind, und
=X für $=N-NO_2$, $=CH-NO_2$ =N-CN oder $=CH-CO-CF_3$ stehen,

EP 0 507 736 A1

in freier Form oder in Säureadditionssalzform, können als agrochemische Wirkstoffe verwendet werden und sind in an sich bekannter Weise herstellbar.

Die vorliegende Erfindung betrifft neue Derivate von Amidinen, Verfahren zu ihrer Herstellung, Schädlingsbekämpfungsmittel, welche diese Verbindungen enthalten, sowie ihre Verwendung bei der Kontrolle von Schädlingen.

Die erfindungsgemässen Amidinderivate entsprechen der Formel

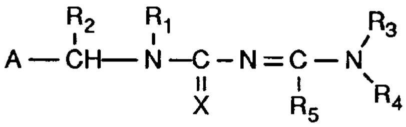
(I),

worin

$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl und
$R_4$ für $C_1$-$C_4$-Alkyl, $C_3$-$C_6$-Cycloalkyl oder $-CH_2CH_2COOR_7$ oder
$R_3$ und $R_4$ gemeinsam für $-(CH_2)_4-$ oder $-(CH_2)_5-$,
$R_5$ für Wasserstoff, $C_1$-$C_4$-Alkyl,

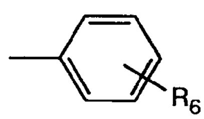
, ·

$-CH_2OCH_3$, -CN, $-COOR_7$ oder

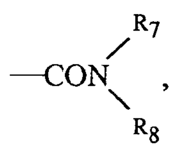
,

$R_5$ für Wasserstoff, Chlor, Methyl oder Nitro,
$R_7$ und $R_5$ für Methyl oder Ethyl,
A für einen unsubstituierten oder ein- bis vierfach substituierten, aromatischen oder nicht-aromatischen, monocyclischen oder bicyclischen, heterocyclischen Rest, wobei ein bis zwei Substituenten aus der Gruppe, bestehend aus $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyano und Nitro, und ein bis vier Substituenten aus der Gruppe, bestehend aus $C_1$-$C_3$-Alkyl, $C_1$-$C_3$-Alkoxy und Halogen, ausgewählt sind, und =X für $=N-NO_2$, $=CH-NO_2$, =N-CN oder $=CH-CO-CF_3$ stehen.

Aus der Literatur sind insektizid wirksame heterocyclische Verbindungen mit verschiedenen zusätzlichen Strukturmerkmalen bekannt. In EP-A 0 375 907 werden einfach ungesättigte insektizide Aktivsubstanzen der Formel

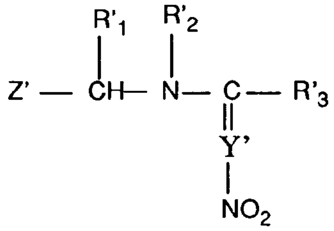

worin Y′ CH oder N, Z′ einen gegebenenfalls $C_1$-$C_4$-alkyl- oder halogensubstituierten heterocyclischen Rest, $R'_1$ und $R'_2$ Wasserstoff oder $C_1$-$C_4$-Alkyl, $R'_3$ $-SR'_4$ oder

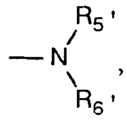,

$R'_4$ $C_1$-$C_4$-Alkyl und $R'_5$ und $R'_6$ Wasserstoff oder $C_1$-$C_4$-Alkyl bedeuten, beschrieben.

In EP-A 0 383 091 sind analoge Verbindungen mit insektizider Aktivität beschrieben, wobei die Bedeutungen der Reste Z′ und $R'_1$ bis $R'_6$ erweitert bzw. verallgemeinert sind.

Die in EP-A 0 376 279 beschriebenen Guanidinderivate der Formel

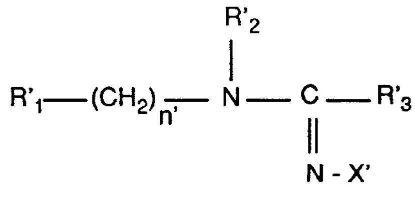

worin $R'_1$ einen gegebenenfalls substituierten Heterocyclus, n′O oder 1, $R'_2$ Wasserstoff oder einen gegebenenfalls substituierten Kohlenwasserstoffrest, $R'_3$ eine Aminogruppe und X′ Cyano bedeutet, sind ebenfalls insektizid wirksam.

Im Unterschied zu den vorstehend erläuterten literaturbekannten Strukturen weisen die erfindungsgemässen Verbindungen I einen mit einer Aminogruppe substituierten Alkylidenrest an einem der Stickstoffatome der Guanidin- oder Enamin-Grundstruktur auf.

Schliesslich weisen die in EP-A 0 302 389 beschriebenen α-ungesättigten Amine der Formel

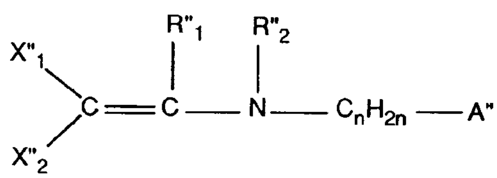

eine insektizide-akarizide Wirksamkeit auf, wobei A″ als heterocyclischer Rest oder cyclischer Kohlenwasserstoffrest, $R''_1$ als eine "über ein Stickstoffatom gebundene Gruppe", $R''_2$ als Wasserstoff oder "eine über ein Kohlenstoff-, Stickstoff- oder Sauerstoffatom gebundene Gruppe", einer der Reste $X''_1$ und $X''_2$ als elektronenanziehende Gruppe, der andere der Reste $X''_1$ und $X''_2$ als Wasserstoff oder elekronenanziehende Gruppe und n als 0, 1 oder 2 definiert werden. Verbindungen mit einer Amidinstruktur, wie sie die erfindungsgemässen Verbindungen I aufweisen, werden jedoch in EP-A 0 302 389 nicht beschrieben.

Die biologischen Eigenschaften der in den vorstehend erwähnten Patentanmeldungen beschriebenen Verbindungen vermögen auf dem Gebiet der Schädlingsbekämpfung nicht voll zu befriedigen, weshalb das Bedürfnis besteht, weitere Verbindungen mit schädlingsbekämpfenden Eigenschaften zur Verfügung zu stehen, wobei diese Aufgabe erfindungsgemäss durch die Bereitstellung der vorliegenden Verbindungen I gelöst wird.

Die erfindungsgemässen Verbindungen I schliessen auch, insbesondere agrochemisch verträgliche, Säureadditionssalze ein. Beispiele geeigneter (anorganischer oder organischer) Säuren sind Salzsäure, Bromwasserstoffsäure, Schwefelsäure, Phosphorsäure, Salpetersäure, Säuren mit gleichem Zentralatom und höherer oder niedrigerer Oxidationsstufe, wie Perchlorsäure, salpetrige Säure oder phosphorige Säure, Essigsäure und Bernsteinsäure.

Die in der Definition des heterocyclischen Restes A eingeschlossenen Ringsysteme enthalten als Ringglied mindestens ein Heteroatom; mindestens eines der den zugrundeliegenden ringförmigen Grundkörper bildenden Atome ist also von Kohlenstoff verschieden. Grundsätzlich sind diejenigen Atome des periodischen Systems der Elemente befähigt, als von Kohlenstoff verschiedene Ringglieder zu fungieren, die mindestens zwei kovalente Bindungen bilden können. Der heterocyclische Rest A ist dabei bevorzugt ungesättigt und über ein Kohlenstoff-Ringglied an das den Rest $R_2$ tragende Kohlenstoffatom der Formel I gebunden. Ungesättigte Ringsysteme A enthalten eine oder mehrere Doppelbindungen; vorzugsweise sind derartige Ringsysteme A mehrfach ungesättigt und weisen im allgemeinen einen aromatischen Charakter auf. Bevorzugt sind solche Ringsysteme A, welche mindestens ein Stickstoffatom enthalten. Üblicherweise enthalten solche Ringe A ein

bis drei Heteroatome aus der Gruppe, bestehend aus Sauerstoff, Schwefel und Stickstoff, wobei jeweils höchstens eines der Heteroatome ein Sauerstoff- oder ein Schwefelatom ist. Bevorzugt sind Ringsysteme A, die ein bis drei Heteroatome aus der Gruppe, bestehend aus Sauerstoff, Schwefel und Stickstoff, enthalten, wobei stets mindestens ein Stickstoffatom in A enthalten ist und höchstens eines der gegebenenfalls weiteren in A enthaltenen Heteroatome ein Sauerstoffatom oder ein Schwefelatom ist. Insbesondere finden sich Beispiele für erfindungsgemässe Heterocyclen A in der Gruppe, bestehend aus den Grundkörpern

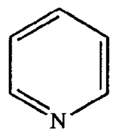 , 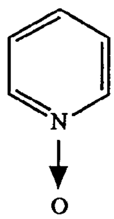 , 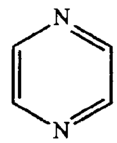 , 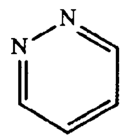 , 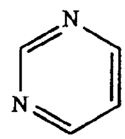 , 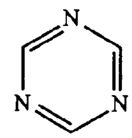 , 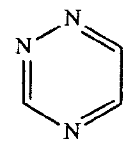 ,

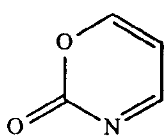 , 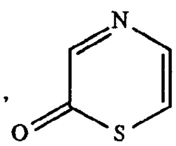 , 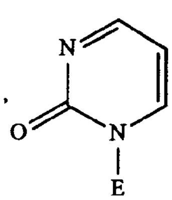 , 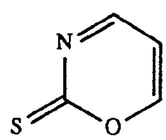 , 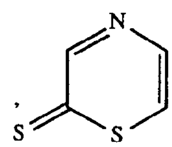 , 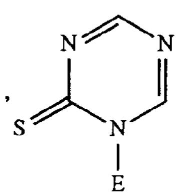 ,

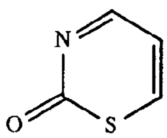 , 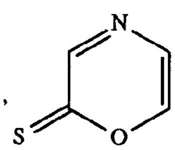 , 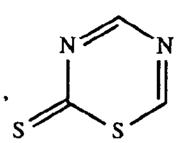 , , 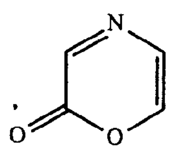 ,

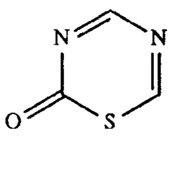, 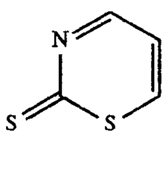, 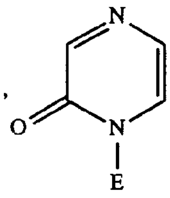, 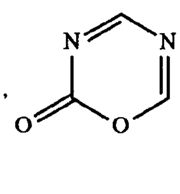, 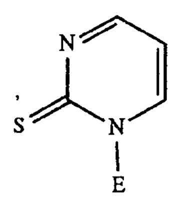, 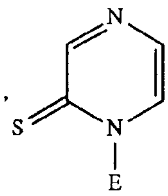,

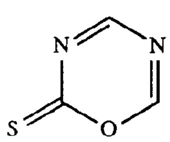, 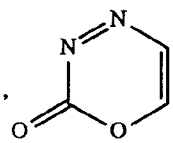, 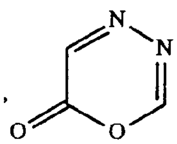, 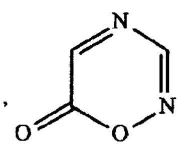, 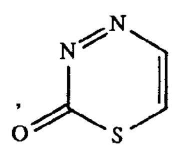,

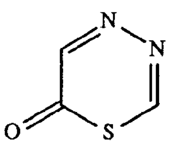, 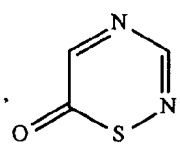, 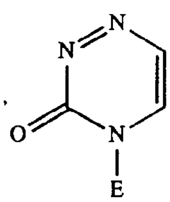, 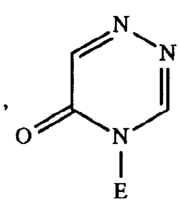, 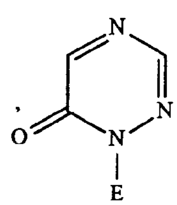,

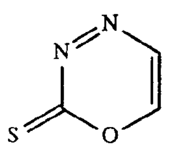, 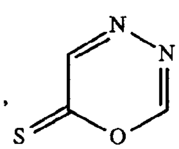, 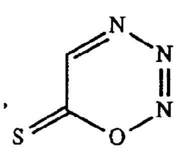, 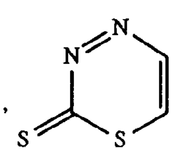, 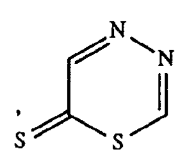,

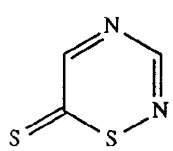, 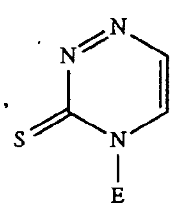, 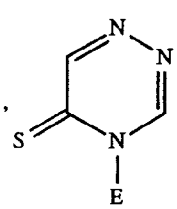, 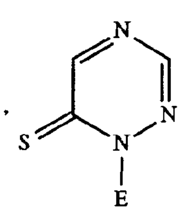, 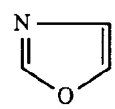,

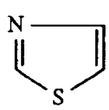, 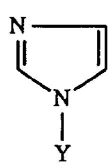, 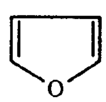, 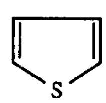,

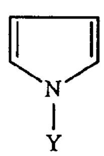, 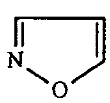, 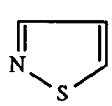, 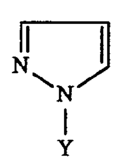,

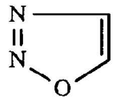 , 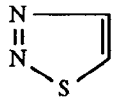 , 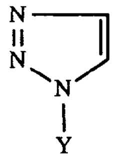 , 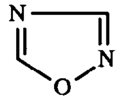 , 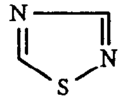 ,

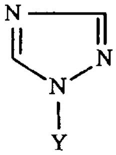 , 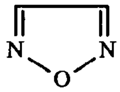 , 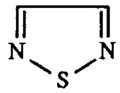 , 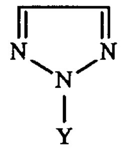 , 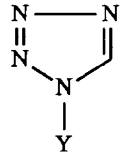 ,

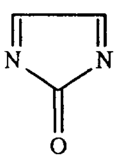 , 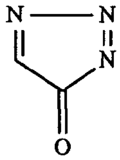 , 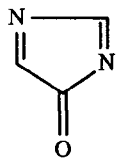 , 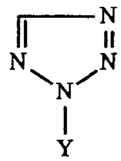 , 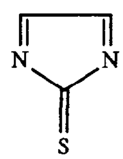 ,

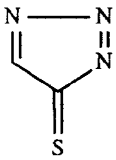 , 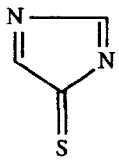 , 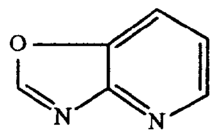 , 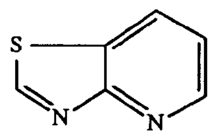 , 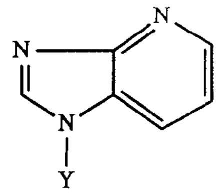 ,

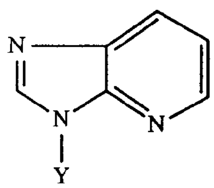 , 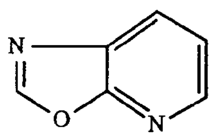 , 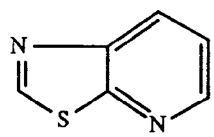 , 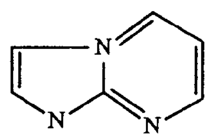 ,

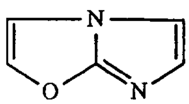 , 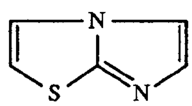 , 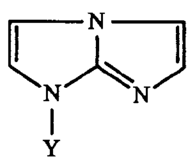 ,

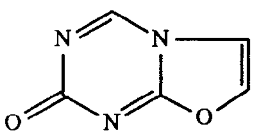 , 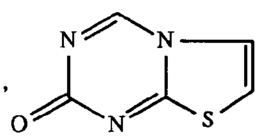 , 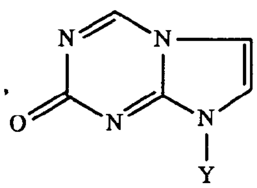 , 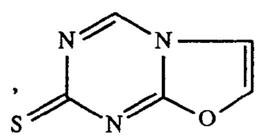 ,

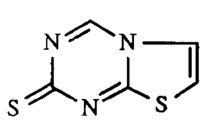

und

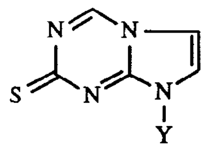 ,

welche unsubstituiert sind oder je nach den Substitutionsmöglichkeiten des Ringsystems bis zu vier der in Anspruch 1 definierten Substituenten tragen, worin E für $C_1$-$C_3$-Alkyl und Y für Wasserstoff, $C_1$-$C_3$-Alkyl oder Cyclopropyl stehen und welche über ein Kohlenstoffatom des Heterocyclus an das den Rest $R_2$ tragende Kohlenstoffatom der Formel I gebunden sind.

Bevorzugt sind diese Heterocyclen A unsubstituiert oder sie tragen ein oder zwei Substituenten, ausgewählt aus der Gruppe, bestehend aus Halogen, $C_1$-$C_3$-Alkyl, $C_1$-$C_3$-Halogenalkyl, $C_1$-$C_3$-Halogenalkoxy und $C_1$-$C_3$-Alkoxy. Ganz besonders bevorzugte Gruppen A leiten sich von Pyridin und Thiazol ab, wie Pyrid-3-yl, 2-Halogenpyrid-5-yl, 2,3-Dihalogenpyrid-5-yl, 2-Halogen-1-oxido-5-pyridinio und 2-Halogenthiazol-5-yl, insbesondere 2-Chlorpyrid-5-yl.

Von den Verbindungen I werden jene bevorzugt, in welchen =X für =N-$NO_2$, =N-CN oder =CH-$NO_2$ steht.

Von diesen Verbindungen I sind jene hervorzuheben, in welchen
$R_1$ für Wasserstoff, $C_1$-$C_3$-Alkyl oder Cyclopropyl,
$R_2$ für Wasserstoff oder Methyl,
$R_3$ für $C_1$-$C_3$-Alkyl und
$R_4$ für $C_1$-$C_3$-Alkyl oder Ethoxycarbonylethyl oder
$R_3$ und $R_4$ gemeinsam für $-(CH_2)_4-$ oder $-(CH_2)_5-$ und
$R_5$ für Wasserstoff, Methyl, Ethyl, Phenyl $-CH_2OCH_3$, -CN, $-COOC_2H_5$ oder

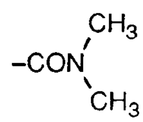

stehen. Von letzteren werden jene bevorzugt, in welchen
$R_1$ für Methyl, Ethyl oder Cyclopropyl,
$R_2$ für Wasserstoff,
$R_3$ für Methyl,
$R_4$ für Methyl und
$R_5$ für Wasserstoff oder Methyl stehen. Insbesondere werden von diesen Verbindungen jene bevorzugt, in welchen =X für =N-$NO_2$ oder =CH-$NO_2$ steht, vor allem jene, in welchen =X für =N-$NO_2$ steht.

Als bevorzugte Einzelverbindungen der Formel I sind zu nennen:

(a) N-(2-Chlorpyrid-5-ylmethyl)-N-methyl-N′-(N,N-dimethylaminomethylen)-N″-nitro-guanidin,
(b) N-(2-Chlorpyrid-5-ylmethyl)-N-methyl-N′-[1-(N,N-dimethylamino)ethyliden]-N″-nitro-guanidin,
(c) N-Pyrid-3-ylmethyl-N-methyl-N′-(N,N-dimethylaminomethylen)-N″-nitro-guanidin,
(d) N-(2-Chlorpyrid-5-ylmethyl)-N-methyl-N′-(N,N-dimethylaminomethylen)-N″-cyano-guanidin,
(e) 1-[N-(2-Chlorpyrid-5-ylmethyl)-N-methyl-amino]-1-(N,N-dimethylaminomethylenamino)-2-nitro-äthen,
(f) 1-[N-(2-Chlorpyrid-5-ylmethyl)-N-ethyl-amino]-1-(N,N-dimethylaminomethylenamino)-2-nitro-äthen,
(g) 1-[N-(2-Chlorpyrid-5-ylmethyl)-N-cyclopropyl-amino]-1-(N,N-dimethylaminomethylenamino)-2-nitro-äthen,
(h) 1-[N-(2-Chlorthiazol-5-ylmethyl)-N-methyl-amino]-1-(N,N-dimethylaminomethylenamino)-2-nitro-äthen,
(i) 1-[N-(2-Chlorthiazol-5-ylmethyl)-N-ethyl-amino]-1-(N,N-dimethylaminomethylenamino)-2-nitro-äthen,
(j) 1-[N-(2-Chlorthiazol-5-ylmethyl)-N-cyclopropyl-amino]-1-(N,N-dimethylaminomethylenamino)-2-nitro-äthen,
(k) 1-[N-(2-Chlor-1-oxido-5-pyridiniomethyl)-N-methyl-amino]-1-(N,N-dimethylaminomethylenamino)-2-nitro-äthen,
(l) 1-[N-(2-Chlor-1-oxido-5-pyridimomethyl)-N-ethyl-amino]-1-(N,N-dimethylaminomethylenamino)-2-nitro-äthen,
(m) 1-[N-(2-Chlor-1-oxido-5-pyridiniomethyl)-N-cyclopropyl-amino]-1-(N,N-dimethylaminomethylenamino)-2-nitro-äthen,

(n) N-(2-Chlorthiazol-5-ylmethyl)-N-methyl-N′-(N,N-dimethylaminomethylen)-N″-nitro-guanidin,
(o) N-(2-Chlorthiazol-5-ylmethyl)-N-ethyl-N′-(N,N-dimethylamiomethylen)-N″-nitro-guanidin,
(p) N-(2-Chlorthiazol-5-ylmethyl)-N-cyclopropyl-N′-(N,N-dimethylaminomethylen)-N″-nitro-guanidin,
(q) N-(2-Chlorpyrid-5-ylmethyl)-N-ethyl-N′-(N,N-dimethylaminomethylen)-N″-nitro-guanidin und
(r) N-(2-Chlorpyrid-5-ylmethyl)-N-cyclopropyl-N′-(N,N-dimethylaminomethylen)-N″-nitro-guanidin.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Iod, wobei Fluor, Chlor und Brom bevorzugt sind. Halogen ist dabei als selbständiger Substituent oder als Teil eines Substituenten zu verstehen, wie in Halogenalkyl, Halogenalkylthio, Halogenalkoxy, Halogencyclopropyl, Halogenalkenyl, Halogenalkinyl, Halogenalkyloxy oder Halogenallylthio. Die als Substituenten in Betracht kommenden (Halogen)Alkyl-, (Halogen)Alkylthio-, (Halogen)Alkenyl-, (Halogen)Alkinyl- und (Halogen)Alkoxyreste können geradkettig oder verzweigt sein. Als Beispiele solcher Alkylreste seien Methyl, Aethyl, Propyl, Isopropyl, Butyl, Isobutyl, sek.-Butyl und tert.-Butyl genannt. Als geeignete Alkoxyreste seien genannt: Methoxy, Aethoxy, Propoxy und Isopropoxy. Alkylthio steht für Methylthio, Aethylthio, Isopropylthio und Propylthio. Halogensubstituierte Gruppen, wie Halogenalkyl, können teilweise halogeniert oder perhalogeniert sein. Beispiele der Alkylelemente dieser halogensubstituierten Gruppen sind das ein- bis dreifach durch Fluor, Chlor und/oder Brom substituierte Methyl, wie $CHF_2$ oder $CF_3$; das ein- bis fünffach durch Fluor, Chlor und/oder Brom substituierte Aethyl, wie $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl, wie $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren, wie $CF(CF_3)CHFCF_3$ oder $CH_2(CF_2)_2CF_3$. Weiterhin seien beispielhaft erwähnt: 2-Chlorcyclopropyl, 2,2-Difluorcyclopropyl, 2,2-Difluorvinyl, 2,2-Dichlorvinyl, 2-Chlorallyl, 2,3-Dichlorallyl und 2,3-Dibromallyl.

Bei den Cycloalkylresten handelt es sich um Cyclopropyl, Cyclobutyl, Cyclopentyl und Cyclohexyl. Alkenyl- und Alkinylgruppen enthalten eine ungesättigte Kohlenstoff-Kohlenstoff-Bindung. Typische Vertreter sind Allyl und Propargyl, aber auch Vinyl und Äthinyl.

Die erfindungsgemässen Verbindungen der Formel I können in Analogie zu bekannten Verfahren hergestellt werden, beispielsweise indem man

a) eine Verbindung der Formel

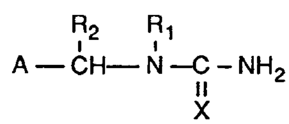

(II),

worin $R_1$, $R_2$, A und X die für die Formel I angegebenen Bedeutungen haben, in einem inerten aprotischen Lösungsmittel mit einer Verbindung der Formel

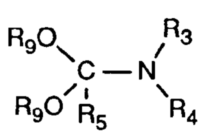

(III),

worin $R_3$, $R_4$ und $R_5$ die für die Formel I angegebenen Bedeutungen haben und $R_9$ $C_1$-$C_4$-Alkyl ist, umsetzt oder

b) eine Verbindung der Formel

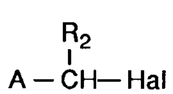

(IV),

worin $R_2$ und A die für die Formel I angegebenen Bedeuningen haben und Hal für Chlor, Brom oder Iod steht, in Gegenwart eines Säureakzeptors in einem Lösungsmittel bei -20 bis +120°C mit einer Verbindung der Formel

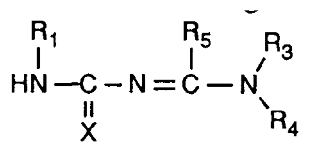

(V),

worin $R_1$, $R_3$, $R_4$, $R_5$ und X die für die Formel I angegebenen Bedeutungen haben, umsetzt und jeweils die entstehende Verbindung der Formel I isoliert oder in ein Säureadditionssalz überführt und dieses isoliert.

Nach einer bevorzugten Ausführungsform der Verfahrensvariante a) wird die Umsetzung in einem inerten aprotischen Lösungsmittel, wie einem Kohlenwasserstoff, einem chlorierten Kohlenwasserstoff, einem Aether, einem Nitril oder einem Amid, bei 20 bis 150°C und unter atmosphärischem Druck ausgeführt. Insbesondere bevorzugt wird die Reaktion bei 50- 120°C in Tetrahydrofuran, Dioxan oder Toluol als Lösungsmittel durchgeführt.

Eine bevorzugte Ausführungsform der Verfahrensvariante b) besteht darin, dass man die Umsetzung in Gegenwart von Natriumhydrid, Natriumamid, Kaliumcarbonat, Triäthylamin oder Diäthylanilin als Säureakzeptor und in N,N-Dimethylformamid,
1-Methylpyrrolidon, Dimethylsulfoxid, Sulfolan, Tetrahydroran oder Acetonitril als Lösungsmittel durchführt. Insbesondere bevorzugt wird die Reaktion mit Natriumhydrid oder Natriumamid als Säureakzeptor bei -10 bis +30°C durchgeführt.

Die Verbindungen der Formeln II, III, IV und V sind bekannt oder können in Analogie zu bekannten Verfahren hergestellt werden. Die Verbindungen der Formel V sind neu und sind auch ein Gegenstand der Erfindung; sie können in Analoge zu bekannten Verfahren hergestellt werden, z.B. wie in den Herstellungsbeispielen beschrieben oder nach analogen Methoden. Bevorzugt sind diejenigen Verbindungen V, in denen die Variablen dieselben Bedeutungen haben wie in den bevorzugten Verbindungen I.

Die erfindungsgemässen Verbindungen I sind bei günstiger Warmblüter-, Fisch- und Pflanzenverträglichkeit wertvolle Wirkstoffe auf dem Gebiet der Schädlingsbekämpfung. Insbesondere wirken die erfindungsgemässen Wirkstoffe gegen Insekten und Spinnentiere, wie sie an Nutz- und Zierpflanzen in der Landwirtschaft und im Gartenbau, insbesondere in Reis-, Baumwoll-, Gemüse- und Obstpflanzungen, und im Forst vorkommen. Weitere Anwendungsgebiete der erfindungsgemässen Wirkstoffe sind der Vorrats- und Materialschutz sowie im Hygienesektor insbesondere der Schutz von Haus- und Nutztieren. Die Verbindungen I sind gegen alle oder einzelne Entwicklungsstadien von normal sensiblen, aber auch von resistenten Arten von Schädlingen wirksam. Dabei kann sich ihre Wirkung z.B. in einer Abtötung der Schädlinge, welche unmittelbar oder erst nach einiger Zeit, beispielsweise bei einer Häutung, eintritt, oder in einer verminderten Eiablage und/oder Schlupfrate zeigen.

Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung Orthoptera zum Beispiel
Blatta spp., Blattella spp., Gryllotalpa spp., Leucophaea maderae, Locusta spp., Periplaneta spp. und Schistocerca spp.;
aus der Ordnung Isoptera zum Beispiel

Reticulitermes spp.;
aus der Ordnung Psocoptera zum Beispiel
Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und Scirtothrips aurantii:
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp.
und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis und
aus der Ordnung Thysanura zum Beispiel
Lepisma saccharina.

Die gute pestizide Wirkung der erfindungsgemässen Verbindungen I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden wesentlich verbreitem und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe- und Bacillus thuringiensis-Präparate.

Die Verbindungen I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln und Granulaten, auch zu Verkapselungen in polymeren Stoffen, in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen I auch für den Einsatz bei der Behandlung von Saatgut Dabei kann sowohl das Saatgut vor dem Säen mit dem Wirkstoff oder einer den Wirkstoff enthaltenden Formulierung behandelt oder gebeizt werden, als auch der Wirkstoff beim Säen in die Saatfurche appliziert werden.

Die Formulierungen, das heisst die den Wirkstoff der Formel I, beziehungsweise eine Kombination dieses Wirkstoffs mit anderen Insektiziden, und gegebenenfalls feste oder flüssige Hilfsstoffe enthaltenden Mittel, Zubereitungen oder Zusammensetzungen, werden in bekannter Weise hergestellt, zum Beispiel durch inniges Vermischen und/oder Vermahlen des Wirkstoffs mit Streckmitteln, wie Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen, wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetanolalkohol, stark polare Lösungsmittel, wie N-Methyl-2-pyrrolidon, Dimethylsulfoxid oder N,N-Dimethylformamid, Wasser sowie Pflanzenöle, wie Raps-, Rizinus-, kokosnuss- oder Sojaöl; gegebenenfalls auch Silikonöle.

Als feste Trägerstoffe, beispielsweise für Stäubemittel und dispergerbare Pulver, werden in der Regel natürliche Gesteinsmehle verwendet, wie Calcit, Talkum, Kaolin, Montmorillonit oder Attapulgt. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen je nach Art des zu formulierenden Wirkstoffes der Formel I oder der Kombination dieses Wirkstoffs mit anderen Insektiziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$, wie die Natrium- oder Kalium-Salze der Oel- oder Stearinsäure oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate. Die Fettsulfonate oder -sulfate liegen in der Regel als Alkali-, Erdalkali- oder gegebenenfalls substituierte Ammoniumsalze vor und weisen im allgemeinen einen Alkyrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolammoniumsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie Salze des Phosphorsäureesters eines p-Nonylphenol-(4-14)-Aethylenoxid-Adduktes oder Phospholipide, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat, in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, Beispiele sind das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:

"Mc Cutcheon's Detergents and Emulsifiers Annual", Mc Publishing Corp., Glen Rock, NJ, USA, 1988";

H. Stache, "Tensid-Taschenbuch", 2. Aufl., C. Hanser Verlag, München, Wien, 1981 und M. and J. Ash. "Encyclopedia of Surfactants", Vol. I-III, Chemical Publishing Co., New York, 1980- 1981.

Die pestiziden Zubereitungen enthalten in der Regel 0,1 bis 99 %, insbesondere 0,1 bis 95 % Wirkstoff der Formel I oder der Kombination dieses Wirkstoffs mit anderen Insektiziden, 1 bis 99,9 % eines festen oder

flüssigen Hilfsstolles und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent):

Emulgierbare Konzentrate:

| | |
|---|---|
| Wirkstoff: | 1 bis 90 %, bevorzugt 5 bis 20 % |
| oberflächenaktives Mittel: | 1 bis 30 %, vorzugsweise 10 bis 20 % |
| flüssiges Trägermaterial: | 5 bis 94 %, vorzugsweise 70 bis 85 % |

Stäube:

| | |
|---|---|
| Wirkstoff: | 0,1 bis 10 %, vorzugsweise 0,1 bis 1 % |
| festes Trägermaterial: | 99,9 bis 90 %, vorzugsweise 99,9 bis 99 % |

Suspension-Konzentrate:

| | |
|---|---|
| Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Wirkstoff: | 0,5 bis 30 %, vorzugsweise 3 bis 15 % |
| festes Trägermaterial: | 99,5 bis 70 %, vorzugsweise 97 bis 85 % |

Die Mittel können auch weitere Zusätze, wie Stabilisatoren, z.B. gegebenenfalls epoxidierte Pflanzenöle (z.B. epoxidiertes Kokosnussöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein. Temperaturen sind in Grad Celsius angegeben.

Herstellungsbeispiele

Beispiel 1: N-(2-Chlorpyrid-5-ylmethyl)-N-methyl-N′-(N,N-dimethylaminomethylen)-N″-nitro-guanidin (Tabelle 1, Verbindung Nr. 1.001).

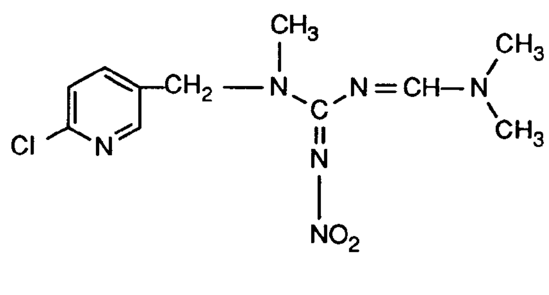

Variante a): Zu 2,43 g N-(2-Chlorpyrid-5-ylmethyl)-N-methyl-N′-nitro-guanidin in 75 ml Dioxan werden 1,5 g N,N-Dimethylformamiddiäthylacetal zugegeben. Das Gemisch wird 45 Minuten auf 130°C erhitzt und dann

am Rotationsverdampfer vom Lösungsmittel befreit. Das Rohprodukt wird an Kieselgel zuerst mit Aethylacetat und dann mit Aethylacetat/Aethanol (5:1) chromatographiert. Es resultiert ein zähes Oel, das nach Versetzen mit Hexan Kristalle des gewünschten Produkts (Smp.: 85-89°C) liefert.

Variante b): 0,6 g Natriumhydrid (80 %ig, in Mineralöl) werden unter Stickstoffatmosphäre in kleinen Portionen zu 3,46 g N-(N,N-Dimethylaminomethylen)-N'-methyl-N''-nitro-guanidin in 35 ml absolutem N,N-Dimethylformamid zugefügt. Nach einstündigem Rühren bei 5°C wird eine Lösung von 3,24 g 2-Chlor-5-chlormethyl-pydin in 10 ml N,N-Dimethylformamid zugetropft und das Reakionsgemisch über Nacht bei 20°C gerührt. Nach dem Abfiltrieren wird das Filtrat im Hochvakuum eingeengt und das erhaltene Oel durch Säulenchromatographie ($SiO_2$/Aethylacetat) gereinigt. Das gewünschte Produkt ist ein weisses Pulver (Smp.: 90-94°C).

Beispiel 2: 1-[N-(2-Chlorpyrid-5-ylmethyl)-N-methyl-amino]-1-(N,N-dimethylaminomethylenamino)-2-nitro-äthen (Tabelle 5, Verbindung Nr. 5.001).

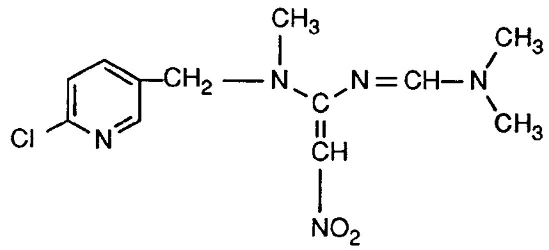

Variante a): Ein Gemisch aus 0,9 g 1-Amino-1-[N-(2-chlorpyrid-5-ylmethyl)-N-methylamino]-2-nitro-äthen, 0,68 ml N,N-Dimethylformamiddiäthylacetal und 20 ml 1,3-Dioxan wird unter einer Argonatmosphäre 10 Stunden unter Rückfluss erhitzt. Nach Abdestillieren des Lösungsmittels wird der Rückstand mit Diäthyläther versetzt und das kristalline Produkt abfiltriert (Smp.: 108-110°C).

Variante b1): Zu einem Gemisch aus 1,0 g 1-(N,N-Dimethylaminomethylenamino)-1-(N-methylamino)-2-nitro-äthen, 1,13 g 2-Chlor-5-chlormethyl-pyridin und 10 ml N,N-Dimethylformamid werden 2,01 g Kaliumcarbonat zugegeben. Anschliessend wird das Reaktionsgemisch 2 Tage bei 50°C gerührt. Nach dem Abfiltrieren wird das Filtrat im Hochvakuum eingeengt und das erhaltene Rohprodukt durch Säulenchromatographie [$SiO_2$; $CH_2Cl_2$/MeOH (10:1)] gereinigt. Man erhält das gewünschte Produkt, das bei 107-110°C schmilzt.

Variante b2): Zu einer Suspension von 1,0 g 1-(N,N-Dimethylaminomethylenamino)-1-(N-methylamino)-2-nitro-äthen in 20 ml N,N-Dimethylformamid werden 0,19 g Natriumhydrid (80%ig, in Mineralöl) zugegeben. Nach sechsstündigem Rühren bei Raumtemperatur werden 1,13 g 2-Chlor-5-chlormethyl-pyridin zugegeben. Das Reaktionsgemisch wird anschliessend über Nacht bei Raumtemperatur gerührt. Nach dem Abfiltrieren wird das Filtrat im Hochvakuum eingeengt und das erhaltene Rohprodukt durch Säulenchromatographie [$SiO_2$; $CH_2Cl_2$/MeOH (10:1)] gereinigt. Man erhält das gewünschte Produkt, das bei 108-110°C schmilzt.

Beispiel 3: N-Cyclopropyl-N'-(N,N-dimethylaminomethylen)-N''-nitro-guanidin (Herstellungsbeispiel für Verbindungen der Formel V).

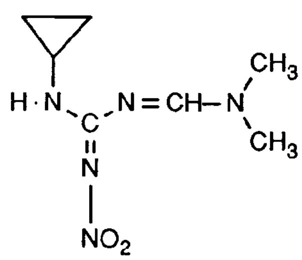

a) 140 g S-Methylisothioharnstoffsulfat werden bei -10°C in Portionen innerhalb einer Stunde zu einem Gemisch von 140 ml rauchender Salpetersäure und 420 ml konzentrierter Schwefelsäure gegeben. Nach Ausrühren des Reaktionsgemisches während einer Stunde bei 0°C wird auf 21 Eis/Wasser gegossen und das weisse Produkt abgesaugt. Der Filterkuchen wird mit 250 ml Wasser, 100 ml kaltem Aethanol und 150 ml Diäthyläther gewaschen und im Vakuum bei Raumtemperatur getrocknet. Man erhält so den S-Methyl-N-nitro-isothioharnstoff (Smp.: 162- 164°C).

b) 27 g S-Methyl-N-nitro-isothioharnstoff werden im Gemisch mit 12,6 g Cyclopropylamin und 15 ml Aethanol 3,5 Stunden unter Rückfluss erhitzt. Dann wird das Reaktionsgemisch auf -5°C gekühlt und das Produkt abfiltriert und mit wenig kaltem Aethanol und Diäthyläther gewaschen. Man erhält so das N-Cyclopropyl-N'-nitro-guanidin (Smp.: 127- 130°C).

c) 14,4 g N-Cyclopropyl-N'-nitro-guanidin werden zu 14,7 g N,N-Dimethylformamiddiäthylacetal zugegeben. Nach 1,5-stündigem Rühren bei 70-75°C wird das Gemisch abgekühlt und am Rotationsverdampfer eingeengt. Das Rohprodukt wird in Diäthyläther verrührt, abfiltriert und mit weiterem Diäthyläther gewaschen. Man erhält so das N-Cyclopropyl-N'-(N,N-dimethylaminomethylen)-N''-nitro-guanidin (Smp.: 120-122°C).

In analoger Weise kann man herstellen
N-Aethyl-N'-(N,N-dimethylaminomethylen)-N''-nitro-guanidin (Smp.: 93-96°C),
N-(N,N-Dimethylaminomethylen)-N'-methyl-N''-nitro-guanidin (Smp.: 141-143°C),
N-Cyclopropyl-N'-[1-(N,N-dimethylamino)ethyliden]-N''-nitro-guanidin (Smp.: 92-94°C)
und
N-Aethyl-N'-[1-(N,N-dimethylamino)ethyliden]-N''-nitro-guanidin (Smp.: 74-77°C).

Beispiel 4: 1-(N,N-Dimethylaminomethylenamino)-1-(N-methylamino)-2-nitro-aethen (Herstellungsbeispiel für Verbindungen der Formel V).

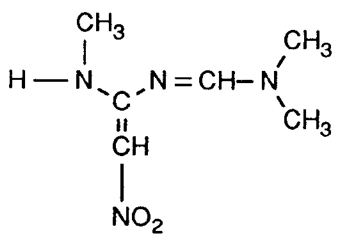

Zu 20 g 1-Amino-1-(N-methylamino)-2-nitro-aethen in 100 ml Dioxan werden 32,2 ml N,N-Dimethylformamiddiaethylacetal zugegeben. Das Reaktionsgemisch wird 16 Stunden auf 50°C erhitzt. Die erhaltenen Kristalle werden abfiltriert und mit Diäthyläther gewaschen. Man erhält so das gesuchte Produkt, das bei 219°C schmilzt.

In analoger Weise kann man herstellen:
1-(N-Aethylamino)-1-(N,N-dimethylaminomethylenamino)-2-nitro-aethen (ausgehend von 1-(N-Aethylamino)-1-amino-2-nitro-aethen) und
1-(N-Cyclopropylamino)-1-(N,N-dimethylaminomethylenamino)-2-nitro-aethen (ausgehend von 1-Amino-1-(N-cyclopropylamino)-2-nitro-aethen).

In analoger Weise wie in den Beispielen 1 bis 4 beschrieben können auch die anderen in den folgenden Tabellen 1 bis 13 aufgeführten Verbindungen der Formel I hergestellt werden. In der Spalte "Schmp." dieser Tabellen bezeichnen die angegebenen Temperaturen jeweils den Schmelzpunkt der betreffenden Verbindung. Gegebenenfalls sind in dieser Spalte "Schmp. " auch andere physikalische Daten aufgeführt, so steht beispielsweise $n_D^T$ für den Brechungsindex der betreffenden Verbindung bei der Temperatur T°C.

Tabelle 1

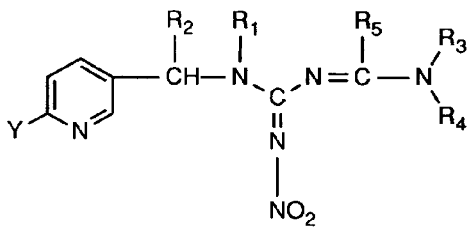

| Verb. Nr. | Y | $R_1$ | $R_2$ | $R_3$ | $R_4$ | $R_5$ | Schmp. |
|---|---|---|---|---|---|---|---|
| 1.001 | Cl | $CH_3$ | H | $CH_3$ | $CH_3$ | H | 85-89°C |
| 1.002 | Cl | H | H | $CH_3$ | $CH_3$ | H | 142-143°C |
| 1.003 | Cl | $CH_3$ | H | $CH_3$ | $CH_3$ | $CH_3$ | 72-76°C |
| 1.004 | Cl | $CH_3$ | H | $CH_3$ | $C_2H_5$ | H | |
| 1.005 | Cl | $CH_3$ | H | $C_2H_5$ | $C_2H_5$ | H | |
| 1.006 | Cl | $CH_3$ | H | $nC_3H_7$ | $nC_3H_7$ | H | |
| 1.007 | Cl | $CH_3$ | H | $C_2H_5$ | $CH_3$ | $CH_3$ | |
| 1.008 | Cl | H | H | $CH_3$ | $CH_3$ | $CH_3$ | |
| 1.009 | Cl | $CH_3$ | H | $C_2H_5$ | $C_2H_5$ | $CH_3$ | |
| 1.010 | Cl | $CH_3$ | $CH_3$ | $CH_3$ | $CH_3$ | H | |
| 1.011 | Cl | $CH_3$ | $CH_3$ | $C_2H_5$ | $C_2H_5$ | H | |
| 1.012 | Cl | $C_2H_5$ | H | $CH_3$ | $CH_3$ | H | 81-84°C |
| 1.013 | Cl | $C_2H_5$ | H | $CH_3$ | $CH_3$ | $CH_3$ | 113-116°C |
| 1.014 | Cl | $C_2H_5$ | H | $CH_3$ | $C_2H_5$ | H | |
| 1.015 | Cl | $C_2H_5$ | H | $C_2H_5$ | $C_2H_5$ | H | |
| 1.016 | Cl | $C_2H_5$ | H | $nC_3H_7$ | $nC_3H_7$ | H | |
| 1.017 | Cl | $C_2H_5$ | H | $CH_3$ | $CH_3$ | $C_2H_5$ | |
| 1.018 | Cl | $C_2H_5$ | H | $C_2H_5$ | $CH_3$ | $CH_3$ | |
| 1.019 | Cl | $C_2H_5$ | H | $C_2H_5$ | $C_2H_5$ | $CH_3$ | |
| 1.020 | Cl | $C_2H_5$ | $CH_3$ | $CH_3$ | $CH_3$ | H | |
| 1.021 | Cl | $C_2H_5$ | $CH_3$ | $C_2H_5$ | $C_2H_5$ | H | Harz |
| 1.022 | Cl | 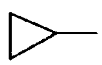 | H | $CH_3$ | $CH_3$ | H | Harz |
| 1.023 | Cl | 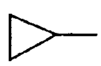 | H | $CH_3$ | $CH_3$ | $CH_3$ | |

Tabelle 1 (Fortsetzung)

| Verb. Nr. | Y | $R_1$ | $R_2$ | $R_3$ | $R_4$ | $R_5$ | Schmp. |
|---|---|---|---|---|---|---|---|
| 1.024 | Cl | 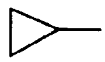 | H | $CH_3$ | $C_2H_5$ | H | |
| 1.025 | Cl | 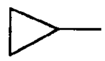 | H | $C_2H_5$ | $C_2H_5$ | H | |
| 1.026 | Cl | 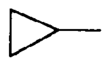 | H | $nC_3H_7$ | $nC_3H_7$ | H | |
| 1.027 | Cl | 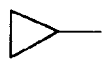 | H | $CH_3$ | $CH_3$ | $C_2H_5$ | |
| 1.028 | Cl | 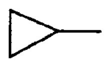 | H | $C_2H_5$ | $CH_3$ | $CH_3$ | |
| 1.029 | Cl | 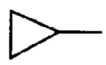 | H | $C_2H_5$ | $C_2H_5$ | $CH_3$ | |
| 1.030 | Cl | 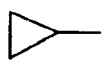 | $CH_3$ | $CH_3$ | $CH_3$ | H | |
| 1.031 | Cl | 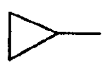 | $CH_3$ | $C_2H_5$ | $C_2H_5$ | H | |
| 1.032 | Cl | $nC_3H_7$ | H | $CH_3$ | $CH_3$ | H | |
| 1.033 | Cl | $nC_3H_7$ | H | $C_2H_5$ | $C_2H_5$ | H | |
| 1.034 | Cl | $CH_3$ | H | $-CH_2CH_2CH_2CH_2-$ | | H | |
| 1.035 | Cl | $CH_3$ | H | $-CH_2CH_2CH_2CH_2CH_2-$ | | H | |
| 1.036 | Cl | H | $CH_3$ | $CH_3$ | $CH_3$ | H | |
| 1.037 | Cl | H | $CH_3$ | $CH_3$ | $CH_3$ | $CH_3$ | |
| 1.038 | H | $CH_3$ | H | $CH_3$ | $CH_3$ | H | 51-54°C |
| 1.039 | H | $CH_3$ | H | $CH_3$ | $CH_3$ | $CH_3$ | |
| 1.040 | H | $CH_3$ | H | $C_2H_5$ | $C_2H_5$ | H | |
| 1.041 | H | H | H | $CH_3$ | $CH_3$ | H | |
| 1.042 | H | $C_2H_5$ | H | $CH_3$ | $CH_3$ | H | |
| 1.043 | H | 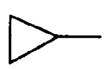 | H | $CH_3$ | $CH_3$ | H | |
| 1.044 | Cl | $CH_3$ | H | $CH_3$ | $CH_3$ | 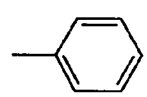 | |
| 1.045 | Cl | $CH_3$ | H | $CH_3$ | $CH_3$ | $-CH_2OCH_3$ | |

Tabelle 1 (Fortsetzung)

| Verb. Nr. | Y | $R_1$ | $R_2$ | $R_3$ | $R_4$ | $R_5$ | Schmp. |
|---|---|---|---|---|---|---|---|
| 1.046 | Cl | $CH_3$ | H | $CH_3$ | $CH_3$ | $-CN$ | |
| 1.047 | Cl | $CH_3$ | H | $CH_3$ | $CH_3$ | $-COOC_2H_5$ | |
| 1.048 | Cl | $CH_3$ | H | $CH_3$ | $CH_3$ | $-CON(CH_3)_2$ | |
| 1.049 | Cl | $CH_3$ | H | $CH_3$ | $-CH_2CH_2COOC_2H_5$ | H | |

Tabelle 2

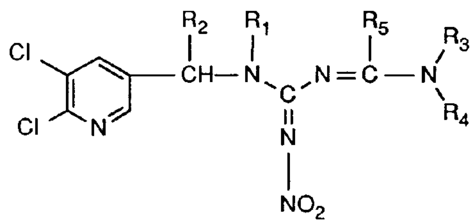

| Verb. Nr. | $R_1$ | $R_2$ | $R_3$ | $R_4$ | $R_5$ | Schmp. |
|---|---|---|---|---|---|---|
| 2.001 | $CH_3$ | H | $CH_3$ | $CH_3$ | H | |
| 2.002 | H | H | $CH_3$ | $CH_3$ | H | |
| 2.003 | $CH_3$ | H | $CH_3$ | $CH_3$ | $CH_3$ | |
| 2.004 | $CH_3$ | H | $CH_3$ | $C_2H_5$ | H | |
| 2.005 | $CH_3$ | H | $C_2H_5$ | $C_2H_5$ | H | |
| 2.006 | $CH_3$ | H | $nC_3H_7$ | $nC_3H_7$ | H | |
| 2.007 | $CH_3$ | H | $C_2H_5$ | $CH_3$ | $CH_3$ | |
| 2.008 | H | H | $CH_3$ | $CH_3$ | $CH_3$ | |
| 2.009 | $CH_3$ | H | $C_2H_5$ | $C_2H_5$ | $CH_3$ | |
| 2.010 | $CH_3$ | $CH_3$ | $CH_3$ | $CH_3$ | H | |
| 2.011 | $CH_3$ | $CH_3$ | $C_2H_5$ | $C_2H_5$ | H | |
| 2.012 | $C_2H_5$ | H | $CH_3$ | $CH_3$ | H | amorph |
| 2.013 | $C_2H_5$ | H | $CH_3$ | $CH_3$ | $CH_3$ | |
| 2.014 | $C_2H_5$ | H | $CH_3$ | $C_2H_5$ | H | |
| 2.015 | $C_2H_5$ | H | $C_2H_5$ | $C_2H_5$ | H | |
| 2.016 | $C_2H_5$ | H | $nC_3H_7$ | $nC_3H_7$ | H | |
| 2.017 | $C_2H_5$ | H | $CH_3$ | $CH_3$ | $C_2H_5$ | |
| 2.018 | $C_2H_5$ | H | $C_2H_5$ | $CH_3$ | $CH_3$ | |
| 2.019 | $C_2H_5$ | H | $C_2H_5$ | $C_2H_5$ | $CH_3$ | |
| 2.020 | $C_2H_5$ | $CH_3$ | $CH_3$ | $CH_3$ | H | |
| 2.021 | $C_2H_5$ | $CH_3$ | $C_2H_5$ | $C_2H_5$ | H | |
| 2.022 | 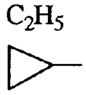 | H | $CH_3$ | $CH_3$ | H | |
| 2.023 | 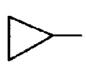 | H | $CH_3$ | $CH_3$ | $CH_3$ | |

Tabelle 2 (Fortsetzung)

| Verb. Nr. | $R_1$ | $R_2$ | $R_3$ | $R_4$ | $R_5$ | Schmp. |
|---|---|---|---|---|---|---|
| 2.024 | 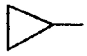 | H | $CH_3$ | $C_2H_5$ | H | |
| 2.025 | 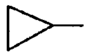 | H | $C_2H_5$ | $C_2H_5$ | H | |
| 2.026 | 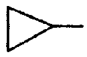 | H | $nC_3H_7$ | $nC_3H_7$ | H | |
| 2.027 | 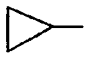 | H | $CH_3$ | $CH_3$ | $C_2H_5$ | |
| 2.028 | 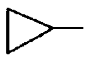 | H | $C_2H_5$ | $CH_3$ | $CH_3$ | |
| 2.029 | 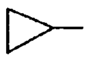 | H | $C_2H_5$ | $C_2H_5$ | $CH_3$ | |
| 2.030 | 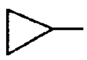 | $CH_3$ | $CH_3$ | $CH_3$ | H | |
| 2.031 | 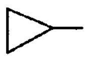 | $CH_3$ | $C_2H_5$ | $C_2H_5$ | H | |
| 2.032 | $nC_3H_7$ | H | $CH_3$ | $CH_3$ | H | |
| 2.033 | $nC_3H_7$ | H | $C_2H_5$ | $C_2H_5$ | H | |
| 2.034 | $CH_3$ | H | $-CH_2CH_2CH_2CH_2-$ | | H | |
| 2.035 | $CH_3$ | H | $-CH_2CH_2CH_2CH_2CH_2-$ | | H | |
| 2.036 | H | $CH_3$ | $CH_3$ | $CH_3$ | H | |
| 2.037 | H | $CH_3$ | $CH_3$ | $CH_3$ | $CH_3$ | |
| 2.038 | $CH_3$ | H | $CH_3$ | $CH_3$ | 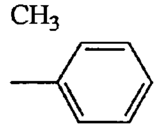 | |
| 2.039 | $CH_3$ | H | $CH_3$ | $CH_3$ | $-CH_2OCH_3$ | |
| 2.040 | $CH_3$ | H | $CH_3$ | $CH_3$ | -CN | |
| 2.041 | $CH_3$ | H | $CH_3$ | $CH_3$ | $-COOC_2H_5-$ | |
| 2.042 | $CH_3$ | H | $CH_3$ | $CH_3$ | 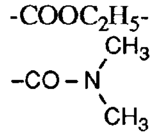 | |
| 2.043 | $CH_3$ | H | $CH_3$ | $CH_2CH_2COOC_2H_5$ | H | |

Tabelle 3

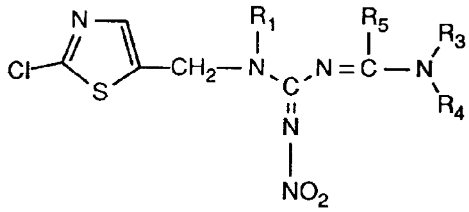

| Verb. Nr. | $R_1$ | $R_4$ | $R_3$ | $R_5$ | Schmp. |
|---|---|---|---|---|---|
| 3.001 | $CH_3$ | $CH_3$ | $CH_3$ | H | 87-90°C |
| 3.002 | H | $CH_3$ | $CH_3$ | H | |
| 3.003 | $CH_3$ | $CH_3$ | $CH_3$ | $CH_3$ | |
| 3.004 | $CH_3$ | $CH_3$ | $C_2H_5$ | H | |
| 3.005 | $CH_3$ | $C_2H_5$ | $C_2H_5$ | H | |
| 3.006 | $CH_3$ | $C_2H_5$ | $CH_3$ | $CH_3$ | |
| 3.007 | $CH_3$ | $C_2H_5$ | $C_2H_5$ | $CH_3$ | |
| 3.008 | $C_2H_5$ | $CH_3$ | $CH_3$ | H | amorph |
| 3.009 | $C_2H_5$ | $CH_3$ | $CH_3$ | $CH_3$ | |
| 3.010 | $C_2H_5$ | $CH_3$ | $C_2H_5$ | H | |
| 3.011 | $C_2H_5$ | $C_2H_5$ | $C_2H_5$ | H | |
| 3.012 | $C_2H_5$ | $nC_3H_7$ | $nC_3H_7$ | H | |
| 3.013 | $C_2H_5$ | $CH_3$ | $CH_3$ | $C_2H_5$ | |
| 3.014 | $C_2H_5$ | $C_2H_5$ | $CH_3$ | $CH_3$ | |
| 3.015 | $C_2H_5$ | $C_2H_5$ | $C_2H_5$ | $CH_3$ | |
| 3.016 | 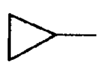 | $CH_3$ | $CH_3$ | H | Oel |
| 3.017 | 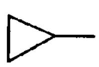 | $CH_3$ | $CH_3$ | $CH_3$ | |
| 3.018 | 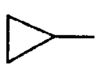 | $CH_3$ | $C_2H_5$ | H | |
| 3.019 | 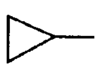 | $C_2H_5$ | $C_2H_5$ | H | |
| 3.020 | 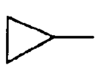 | $nC_3H_7$ | $nC_3H_7$ | H | |
| 3.021 | 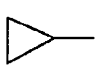 | $C_2H_5$ | $CH_3$ | $CH_3$ | |

Tabelle 3 (Fortsetzung)

| Verb. Nr. | $R_1$ | $R_4$ | $R_3$ | $R_5$ | Schmp. |
|---|---|---|---|---|---|
| 3.022 | 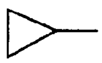 | $C_2H_5$ | $C_2H_5$ | $CH_3$ | |
| 3.023 | $nC_3H_7$ | $CH_3$ | $CH_3$ | H | |
| 3.024 | $nC_3H_7$ | $C_2H_5$ | $C_2H_5$ | H | |
| 3.025 | $CH_3$ | $-CH_2CH_2CH_2CH_2-$ | | H | |
| 3.026 | $CH_3$ | $-CH_2CH_2CH_2CH_2CH_2-$ | | H | |
| 3.027 | H | $CH_3$ | $CH_3$ | $CH_3$ | |
| 3.028 | $CH_3$ | $CH_3$ | $CH_3$ | 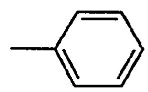 | |
| 3.029 | $CH_3$ | $CH_3$ | $CH_3$ | $-CH_2OCH_3$ | |
| 3.030 | $CH_3$ | $CH_3$ | $CH_3$ | -CN | |
| 3.031 | $CH_3$ | $CH_3$ | $CH_3$ | $-COOC_2H_5$ | |
| 3.032 | $CH_3$ | $CH_3$ | $CH_3$ | $-CON(CH_3)_2$ | |
| 3.033 | $CH_3$ | $CH_3$ | $-CH_2CH_2COOC_2H_5-$ | H | |

Tabelle 4

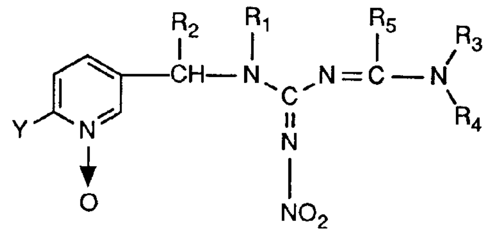

| Verb. Nr. | Y | $R_1$ | $R_2$ | $R_3$ | $R_4$ | $R_5$ | Schmp. |
|---|---|---|---|---|---|---|---|
| 4.001 | Cl | $CH_3$ | H | $CH_3$ | $CH_3$ | H | |
| 4.002 | Cl | H | H | $CH_3$ | $CH_3$ | H | |
| 4.003 | Cl | $CH_3$ | H | $CH_3$ | $CH_3$ | $CH_3$ | |
| 4.004 | Cl | $CH_3$ | H | $C_2H_5$ | $C_2H_5$ | H | |
| 4.005 | Cl | H | H | $CH_3$ | $CH_3$ | $CH_3$ | |
| 4.006 | Cl | $CH_3$ | H | $C_2H_5$ | $C_2H_5$ | $CH_3$ | |
| 4.007 | Cl | $CH_3$ | $CH_3$ | $CH_3$ | $CH_3$ | H | |
| 4.008 | Cl | $C_2H_5$ | H | $CH_3$ | $CH_3$ | H | 52°C |
| 4.009 | Cl | $C_2H_5$ | H | $CH_3$ | $CH_3$ | $CH_3$ | amorph |
| 4.010 | Cl | $C_2H_5$ | H | $C_2H_5$ | $C_2H_5$ | H | |
| 4.011 | Cl | $C_2H_5$ | $CH_3$ | $CH_3$ | $CH_3$ | H | |
| 4.012 | Cl | 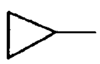 | H | $CH_3$ | $CH_3$ | H | 65°C |
| 4.013 | Cl | 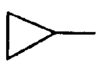 | H | $CH_3$ | $CH_3$ | $CH_3$ | |
| 4.014 | Cl | 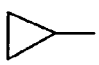 | H | $C_2H_5$ | $C_2H_5$ | H | |
| 4.015 | Cl | 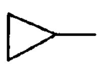 | $CH_3$ | $CH_3$ | $CH_3$ | H | |
| 4.016 | H | $CH_3$ | H | $CH_3$ | $CH_3$ | H | |
| 4.017 | H | $CH_3$ | H | $CH_3$ | $CH_3$ | $CH_3$ | |
| 4.018 | H | $CH_3$ | H | $C_2H_5$ | $C_2H_5$ | H | |
| 4.019 | H | H | H | $CH_3$ | $CH_3$ | H | |
| 4.020 | H | $C_2H_5$ | H | $CH_3$ | $CH_3$ | H | |
| 4.021 | H | 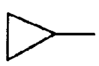 | H | $CH_3$ | $CH_3$ | H | |

Tabelle 4 (Fortsetzung)

| Verb. Nr. | Y | $R_1$ | $R_2$ | $R_3$ | $R_4$ | $R_5$ | Schmp. |
|---|---|---|---|---|---|---|---|
| 4.022 | Cl | $CH_3$ | H | $CH_3$ | $CH_3$ | 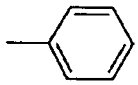 | |
| 4.023 | Cl | $CH_3$ | H | $CH_3$ | $CH_3$ | $-CH_2OCH_3$ | |
| 4.024 | Cl | $CH_3$ | H | $CH_3$ | $CH_3$ | -CN | |
| 4.025 | Cl | $CH_3$ | H | $CH_3$ | $CH_3$ | $-COOC_2H_5$ | |
| 4.026 | Cl | $CH_3$ | H | $CH_3$ | $CH_3$ | $-CON(CH_3)_2$ | |
| 4.027 | Cl | $CH_3$ | H | $CH_3$ | $CH_2CH_2COOC_2H_5$ | H | |

Tabelle 5

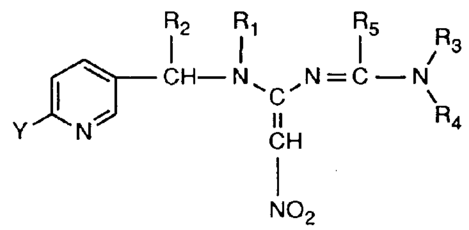

| Verb. Nr. | Y | $R_1$ | $R_2$ | $R_3$ | $R_4$ | $R_5$ | Schmp. |
|---|---|---|---|---|---|---|---|
| 5.001 | Cl | $CH_3$ | H | $CH_3$ | $CH_3$ | H | 108-110°C |
| 5.002 | Cl | H | H | $CH_3$ | $CH_3$ | H | |
| 5.003 | Cl | $CH_3$ | H | $CH_3$ | $CH_3$ | $CH_3$ | |
| 5.004 | Cl | $CH_3$ | H | $CH_3$ | $C_2H_5$ | H | |
| 5.005 | Cl | $CH_3$ | H | $C_2H_5$ | $C_2H_5$ | H | |
| 5.006 | Cl | $CH_3$ | H | $nC_3H_7$ | $nC_3H_7$ | H | |
| 5.007 | Cl | $CH_3$ | H | $C_2H_5$ | $CH_3$ | $CH_3$ | |
| 5.008 | Cl | H | H | $CH_3$ | $CH_3$ | $CH_3$ | |
| 5.009 | Cl | $CH_3$ | H | $C_2H_5$ | $C_2H_5$ | $CH_3$ | |
| 5.010 | Cl | $CH_3$ | $CH_3$ | $CH_3$ | $CH_3$ | H | |
| 5.011 | Cl | $CH_3$ | $CH_3$ | $C_2H_5$ | $C_2H_5$ | H | |
| 5.012 | Cl | $C_2H_5$ | H | $CH_3$ | $CH_3$ | H | 48-50°C |
| 5.013 | Cl | $C_2H_5$ | H | $CH_3$ | $CH_3$ | $CH_3$ | |
| 5.014 | Cl | $C_2H_5$ | H | $CH_3$ | $C_2H_5$ | H | |
| 5.015 | Cl | $C_2H_5$ | H | $C_2H_5$ | $C_2H_5$ | H | |
| 5.016 | Cl | $C_2H_5$ | H | $nC_3H_7$ | $nC_3H_7$ | H | |
| 5.017 | Cl | $C_2H_5$ | H | $CH_3$ | $CH_3$ | $C_2H_5$ | |
| 5.018 | Cl | $C_2H_5$ | H | $C_2H_5$ | $CH_3$ | $CH_3$ | |
| 5.019 | Cl | $C_2H_5$ | H | $C_2H_5$ | $C_2H_5$ | $CH_3$ | |
| 5.020 | Cl | $C_2H_5$ | $CH_3$ | $CH_3$ | $CH_3$ | H | |
| 5.021 | Cl | $C_2H_5$ | $CH_3$ | $C_2H_5$ | $C_2H_5$ | H | |
| 5.022 | Cl | 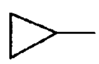 | H | $CH_3$ | $CH_3$ | H | amorph |
| 5.023 | Cl | 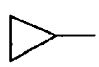 | H | $CH_3$ | $CH_3$ | $CH_3$ | |

Tabelle 5 (Fortsetzung)

| Verb. Nr. | Y | $R_1$ | $R_2$ | $R_3$ | $R_4$ | $R_5$ | Schmp. |
|---|---|---|---|---|---|---|---|
| 5.024 | Cl | 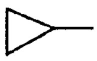 | H | $CH_3$ | $C_2H_5$ | H | |
| 5.025 | Cl | 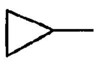 | H | $C_2H_5$ | $C_2H_5$ | H | |
| 5.026 | Cl | 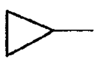 | H | $nC_3H_7$ | $nC_3H_7$ | H | |
| 5.027 | Cl | 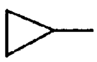 | H | $CH_3$ | $CH_3$ | $C_2H_5$ | |
| 5.028 | Cl | 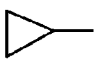 | H | $C_2H_5$ | $CH_3$ | $CH_3$ | |
| 5.029 | Cl | 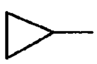 | H | $C_2H_5$ | $C_2H_5$ | $CH_3$ | |
| 5.030 | Cl | 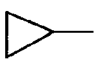 | $CH_3$ | $CH_3$ | $CH_3$ | H | |
| 5.031 | Cl | 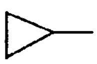 | $CH_3$ | $C_2H_5$ | $C_2H_5$ | H | |
| 5.032 | Cl | $nC_3H_7$ | H | $CH_3$ | $CH_3$ | H | |
| 5.033 | Cl | $nC_3H_7$ | H | $C_2H_5$ | $C_2H_5$ | H | |
| 5.034 | Cl | $CH_3$ | H | $-CH_2CH_2CH_2CH_2-$ | | H | |
| 5.035 | Cl | $CH_3$ | H | $-CH_2CH_2CH_2CH_2CH_2-$ | | H | |
| 5.036 | Cl | H | $CH_3$ | $CH_3$ | $CH_3$ | H | |
| 5.037 | Cl | H | $CH_3$ | $CH_3$ | $CH_3$ | $CH_3$ | |
| 5.038 | H | $CH_3$ | H | $CH_3$ | $CH_3$ | H | |
| 5.039 | H | $CH_3$ | H | $CH_3$ | $CH_3$ | $CH_3$ | |
| 5.040 | H | $CH_3$ | H | $C_2H_5$ | $C_2H_5$ | H | |
| 5.041 | H | H | H | $CH_3$ | $CH_3$ | H | |
| 5.042 | H | $C_2H_5$ | H | $CH_3$ | $CH_3$ | H | |
| 5.043 | H | 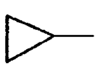 | H | $CH_3$ | $CH_3$ | H | |
| 5.044 | Cl | $CH_3$ | H | $CH_3$ | $CH_3$ | 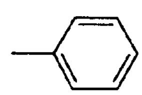 | |
| 5.045 | Cl | $CH_3$ | H | $CH_3$ | $CH_3$ | $-CH_2OCH_3$ | |

Tabelle 5 (Fortsetzung)

| Verb. Nr. | Y | $R_1$ | $R_2$ | $R_3$ | $R_4$ | $R_5$ | Schmp. |
|---|---|---|---|---|---|---|---|
| 5.046 | Cl | $CH_3$ | H | $CH_3$ | $CH_3$ | $-CN$ | |
| 5.047 | Cl | $CH_3$ | H | $CH_3$ | $CH_3$ | $-COOC_2H_5$ | |
| 5.048 | Cl | $CH_3$ | H | $CH_3$ | $CH_3$ | $-CON(CH_3)_2$ | |
| 5.049 | Cl | $CH_3$ | H | $CH_3$ | $-CH_2CH_2COOC_2H_5-$ | H | |

Tabelle 6

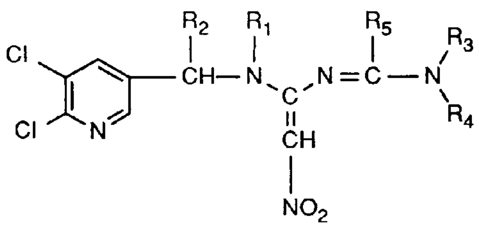

| Verb. Nr. | $R_1$ | $R_2$ | $R_3$ | $R_4$ | $R_5$ | Schmp. |
|---|---|---|---|---|---|---|
| 6.001 | $CH_3$ | H | $CH_3$ | $CH_3$ | H | |
| 6.002 | H | H | $CH_3$ | $CH_3$ | H | |
| 6.003 | $CH_3$ | H | $CH_3$ | $CH_3$ | $CH_3$ | |
| 6.004 | $CH_3$ | H | $CH_3$ | $C_2H_5$ | H | |
| 6.005 | $CH_3$ | H | $C_2H_5$ | $C_2H_5$ | H | |
| 6.006 | $CH_3$ | H | $nC_3H_7$ | $nC_3H_7$ | H | |
| 6.007 | $CH_3$ | H | $C_2H_5$ | $CH_3$ | $CH_3$ | |
| 6.008 | H | H | $CH_3$ | $CH_3$ | $CH_3$ | |
| 6.009 | $CH_3$ | H | $C_2H_5$ | $C_2H_5$ | $CH_3$ | |
| 6.010 | $CH_3$ | $CH_3$ | $CH_3$ | $CH_3$ | H | |
| 6.011 | $CH_3$ | $CH_3$ | $C_2H_5$ | $C_2H_5$ | H | |
| 6.012 | $C_2H_5$ | H | $CH_3$ | $CH_3$ | H | |
| 6.013 | $C_2H_5$ | H | $CH_3$ | $CH_3$ | $CH_3$ | |
| 6.014 | $C_2H_5$ | H | $CH_3$ | $C_2H_5$ | H | |
| 6.015 | $C_2H_5$ | H | $C_2H_5$ | $C_2H_5$ | H | |
| 6.016 | $C_2H_5$ | H | $nC_3H_7$ | $nC_3H_7$ | H | |
| 6.017 | $C_2H_5$ | H | $CH_3$ | $CH_3$ | $C_2H_5$ | |
| 6.018 | $C_2H_5$ | H | $C_2H_5$ | $CH_3$ | $CH_3$ | |
| 6.019 | $C_2H_5$ | H | $C_2H_5$ | $C_2H_5$ | $CH_3$ | |
| 6.020 | $C_2H_5$ | $CH_3$ | $CH_3$ | $CH_3$ | H | |
| 6.021 | $C_2H_5$ | $CH_3$ | $C_2H_5$ | $C_2H_5$ | H | |
| 6.022 | 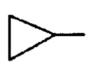 | H | $CH_3$ | $CH_3$ | H | |
| 6.023 | 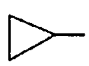 | H | $CH_3$ | $CH_3$ | $CH_3$ | |

Tabelle 6 (Fortsetzung)

| Verb. Nr. | $R_1$ | $R_2$ | $R_3$ | $R_4$ | $R_5$ | Schmp. |
|---|---|---|---|---|---|---|
| 6.024 | 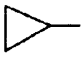 | H | $CH_3$ | $C_2H_5$ | H | |
| 6.025 | 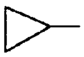 | H | $C_2H_5$ | $C_2H_5$ | H | |
| 6.026 | 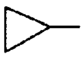 | H | $nC_3H_7$ | $nC_3H_7$ | H | |
| 6.027 | 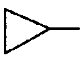 | H | $CH_3$ | $CH_3$ | $C_2H_5$ | |
| 6.028 | 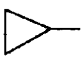 | H | $C_2H_5$ | $CH_3$ | $CH_3$ | |
| 6.029 | 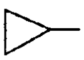 | H | $C_2H_5$ | $C_2H_5$ | $CH_3$ | |
| 6.030 | 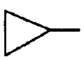 | $CH_3$ | $CH_3$ | $CH_3$ | H | |
| 6.031 | 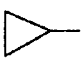 | $CH_3$ | $C_2H_5$ | $C_2H_5$ | H | |
| 6.032 | $nC_3H_7$ | H | $CH_3$ | $CH_3$ | H | |
| 6.033 | $nC_3H_7$ | H | $C_2H_5$ | $C_2H_5$ | H | |
| 6.034 | $CH_3$ | H | $-CH_2CH_2CH_2CH_2-$ | | H | |
| 6.035 | $CH_3$ | H | $-CH_2CH_2CH_2CH_2CH_2-$ | | H | |
| 6.036 | H | $CH_3$ | $CH_3$ | $CH_3$ | H | |
| 6.037 | H | $CH_3$ | $CH_3$ | $CH_3$ | $CH_3$ | |
| 6.038 | $CH_3$ | H | $CH_3$ | $CH_3$ | 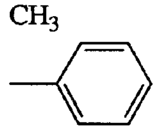 | |
| 6.039 | $CH_3$ | H | $CH_3$ | $CH_3$ | $-CH_2OCH_3$ | |
| 6.040 | $CH_3$ | H | $CH_3$ | $CH_3$ | -CN | |
| 6.041 | $CH_3$ | H | $CH_3$ | $CH_3$ | $-COOC_2H_5$ | |
| 6.042 | $CH_3$ | H | $CH_3$ | $CH_3$ | $-CON(CH_3)_2$ | |
| 6.043 | $CH_3$ | H | $CH_3$ | $CH_2CH_2COOC_2H_5$ | H | |

Tabelle 7

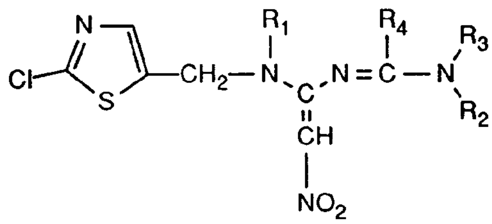

| Verb. Nr. | $R_1$ | $R_2$ | $R_3$ | $R_4$ | Schmp. |
|---|---|---|---|---|---|
| 7.001 | $CH_3$ | $CH_3$ | $CH_3$ | H | amorph |
| 7.002 | H | $CH_3$ | $CH_3$ | H | |
| 7.003 | $CH_3$ | $CH_3$ | $CH_3$ | $CH_3$ | |
| 7.004 | $CH_3$ | $CH_3$ | $C_2H_5$ | H | |
| 7.005 | $CH_3$ | $C_2H_5$ | $C_2H_5$ | H | |
| 7.006 | $CH_3$ | $C_2H_5$ | $CH_3$ | $CH_3$ | |
| 7.007 | $CH_3$ | $C_2H_5$ | $C_2H_5$ | $CH_3$ | |
| 7.008 | $C_2H_5$ | $CH_3$ | $CH_3$ | H | amorph |
| 7.009 | $C_2H_5$ | $CH_3$ | $CH_3$ | $CH_3$ | |
| 7.010 | $C_2H_5$ | $CH_3$ | $C_2H_5$ | H | |
| 7.011 | $C_2H_5$ | $C_2H_5$ | $C_2H_5$ | H | |
| 7.012 | $C_2H_5$ | $nC_3H_7$ | $nC_3H_7$ | H | |
| 7.013 | $C_2H_5$ | $CH_3$ | $CH_3$ | $C_2H_5$ | |
| 7.014 | $C_2H_5$ | $C_2H_5$ | $CH_3$ | $CH_3$ | |
| 7.015 | $C_2H_5$ | $C_2H_5$ | $C_2H_5$ | $CH_3$ | |
| 7.016 | 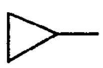 | $CH_3$ | $CH_3$ | H | amorph |
| 7.017 | 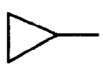 | $CH_3$ | $CH_3$ | $CH_3$ | |
| 7.018 | 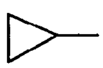 | $CH_3$ | $C_2H_5$ | H | |
| 7.019 | 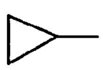 | $C_2H_5$ | $C_2H_5$ | H | |
| 7.020 | 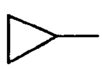 | $nC_3H_7$ | $nC_3H_7$ | H | |
| 7.021 | 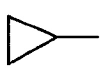 | $C_2H_5$ | $CH_3$ | $CH_3$ | |

Tabelle 7 (Fortsetzung)

| Verb. Nr. | $R_1$ | $R_2$ | $R_3$ | $R_4$ | Schmp. |
|---|---|---|---|---|---|
| 7.022 | 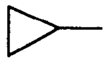 | $C_2H_5$ | $C_2H_5$ | $CH_3$ | |
| 7.023 | $nC_3H_7$ | $CH_3$ | $CH_3$ | H | |
| 7.024 | $nC_3H_7$ | $C_2H_5$ | $C_2H_5$ | H | |
| 7.025 | $CH_3$ | $-CH_2CH_2CH_2CH_2-$ | | H | |
| 7.026 | $CH_3$ | $-CH_2CH_2CH_2CH_2CH_2-$ | | H | |
| 7.027 | H | $CH_3$ | $CH_3$ | $CH_3$ | |
| 7.028 | $CH_3$ | $CH_3$ | $CH_3$ | 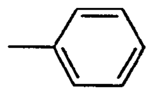 | |
| 7.029 | $CH_3$ | $CH_3$ | $CH_3$ | $-CH_2OCH_3$ | |
| 7.030 | $CH_3$ | $CH_3$ | $CH_3$ | -CN | |
| 7.031 | $CH_3$ | $CH_3$ | $CH_3$ | $-COOC_2H_5$ | |
| 7.032 | $CH_3$ | $CH_3$ | $CH_3$ | $-CON(CH_3)_2$ | |
| 7.033 | $CH_3$ | $CH_3$ | $-CH_2CH_2COOC_2H_5-$ | H | |

Tabelle 8

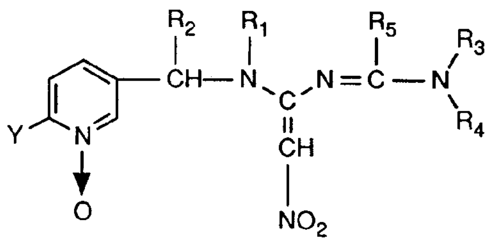

| Verb. Nr. | Y | $R_1$ | $R_2$ | $R_3$ | $R_4$ | $R_5$ | Schmp. |
|---|---|---|---|---|---|---|---|
| 8.001 | Cl | $CH_3$ | H | $CH_3$ | $CH_3$ | H | amorph |
| 8.002 | Cl | H | H | $CH_3$ | $CH_3$ | H | |
| 8.003 | Cl | $CH_3$ | H | $CH_3$ | $CH_3$ | $CH_3$ | |
| 8.004 | Cl | $CH_3$ | H | $C_2H_5$ | $C_2H_5$ | H | |
| 8.005 | Cl | H | H | $CH_3$ | $CH_3$ | $CH_3$ | |
| 8.006 | Cl | $CH_3$ | H | $C_2H_5$ | $C_2H_5$ | $CH_3$ | |
| 8.007 | Cl | $CH_3$ | $CH_3$ | $CH_3$ | $CH_3$ | H | |
| 8.008 | Cl | $C_2H_5$ | H | $CH_3$ | $CH_3$ | H | amorph |
| 8.009 | Cl | $C_2H_5$ | H | $CH_3$ | $CH_3$ | $CH_3$ | |
| 8.010 | Cl | $C_2H_5$ | H | $C_2H_5$ | $C_2H_5$ | H | |
| 8.011 | Cl | $C_2H_5$ | $CH_3$ | $CH_3$ | $CH_3$ | H | |
| 8.012 | Cl | 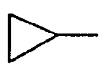 | H | $CH_3$ | $CH_3$ | H | amorph |
| 8.013 | Cl | 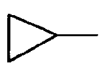 | H | $CH_3$ | $CH_3$ | $CH_3$ | |
| 8.014 | Cl | 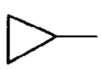 | H | $C_2H_5$ | $C_2H_5$ | H | |
| 8.015 | Cl | 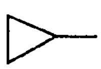 | $CH_3$ | $CH_3$ | $CH_3$ | H | |
| 8.016 | H | $CH_3$ | H | $CH_3$ | $CH_3$ | H | |
| 8.017 | H | $CH_3$ | H | $CH_3$ | $CH_3$ | $CH_3$ | |
| 8.018 | H | $CH_3$ | H | $C_2H_5$ | $C_2H_5$ | H | |
| 8.019 | H | H | H | $CH_3$ | $CH_3$ | H | |
| 8.020 | H | $C_2H_5$ | H | $CH_3$ | $CH_3$ | H | |
| 8.021 | H | 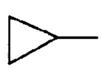 | H | $CH_3$ | $CH_3$ | H | |

Tabelle 8 (Fortsetzung)

| Verb. Nr. | Y | $R_1$ | $R_2$ | $R_3$ | $R_4$ | $R_5$ | Schmp. |
|---|---|---|---|---|---|---|---|
| 8.022 | Cl | $CH_3$ | H | $CH_3$ | $CH_3$ | 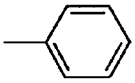 | |
| 8.023 | Cl | $CH_3$ | H | $CH_3$ | $CH_3$ | $-CH_2OCH_3$ | |
| 8.024 | Cl | $CH_3$ | H | $CH_3$ | $CH_3$ | -CN | |
| 8.025 | Cl | $CH_3$ | H | $CH_3$ | $CH_3$ | $-COOC_2H_5$ | |
| 8.026 | Cl | $CH_3$ | H | $CH_3$ | $CH_3$ | $-CON(CH_3)_2$ | |
| 8.027 | Cl | $CH_3$ | H | $CH_3$ | $CH_2CH_2COOC_2H_5$ | H | |

Tabelle 9

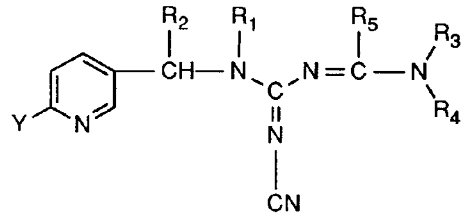

| Verb. Nr. | Y | $R_1$ | $R_2$ | $R_3$ | $R_4$ | $R_5$ | Schmp. |
|---|---|---|---|---|---|---|---|
| 9.001 | Cl | $CH_3$ | H | $CH_3$ | $CH_3$ | H | 94-96°C |
| 9.002 | Cl | H | H | $CH_3$ | $CH_3$ | H | |
| 9.003 | Cl | $CH_3$ | H | $CH_3$ | $CH_3$ | $CH_3$ | $n_D^{23} = 1{,}5559$ |
| 9.004 | Cl | $CH_3$ | H | $CH_3$ | $C_2H_5$ | H | |
| 9.005 | Cl | $CH_3$ | H | $C_2H_5$ | $C_2H_5$ | H | |
| 9.006 | Cl | $CH_3$ | H | $nC_3H_7$ | $nC_3H_7$ | H | |
| 9.007 | Cl | $CH_3$ | H | $C_2H_5$ | $CH_3$ | $CH_3$ | |
| 9.008 | Cl | H | H | $CH_3$ | $CH_3$ | $CH_3$ | |
| 9.009 | Cl | $CH_3$ | H | $C_2H_5$ | $C_2H_5$ | $CH_3$ | |
| 9.010 | Cl | $CH_3$ | $CH_3$ | $CH_3$ | $CH_3$ | H | |
| 9.011 | Cl | $CH_3$ | $CH_3$ | $C_2H_5$ | $C_2H_5$ | H | |
| 9.012 | Cl | $C_2H_5$ | H | $CH_3$ | $CH_3$ | H | |
| 9.013 | Cl | $C_2H_5$ | H | $CH_3$ | $CH_3$ | $CH_3$ | |
| 9.014 | Cl | $C_2H_5$ | H | $CH_3$ | $C_2H_5$ | H | |
| 9.015 | Cl | $C_2H_5$ | H | $C_2H_5$ | $C_2H_5$ | H | |
| 9.016 | Cl | $C_2H_5$ | H | $nC_3H_7$ | $nC_3H_7$ | H | |
| 9.017 | Cl | $C_2H_5$ | H | $CH_3$ | $CH_3$ | $C_2H_5$ | |
| 9.018 | Cl | $C_2H_5$ | H | $C_2H_5$ | $CH_3$ | $CH_3$ | |
| 9.019 | Cl | $C_2H_5$ | H | $C_2H_5$ | $C_2H_5$ | $CH_3$ | |
| 9.020 | Cl | $C_2H_5$ | $CH_3$ | $CH_3$ | $CH_3$ | H | |
| 9.021 | Cl | $C_2H_5$ | $CH_3$ | $C_2H_5$ | $C_2H_5$ | H | |
| 9.022 | Cl | 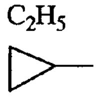 | H | $CH_3$ | $CH_3$ | H | |
| 9.023 | Cl | 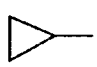 | H | $CH_3$ | $CH_3$ | $CH_3$ | |

Tabelle 9 (Fortsetzung)

| Verb. Nr. | Y | $R_1$ | $R_2$ | $R_3$ | $R_4$ | $R_5$ | Schmp. |
|---|---|---|---|---|---|---|---|
| 9.024 | Cl | 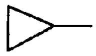 | H | $CH_3$ | $C_2H_5$ | H | |
| 9.025 | Cl | 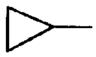 | H | $C_2H_5$ | $C_2H_5$ | H | |
| 9.026 | Cl | 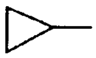 | H | $nC_3H_7$ | $nC_3H_7$ | H | |
| 9.027 | Cl | 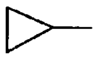 | H | $CH_3$ | $CH_3$ | $C_2H_5$ | |
| 9.028 | Cl | 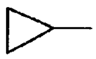 | H | $C_2H_5$ | $CH_3$ | $CH_3$ | |
| 9.029 | Cl | 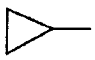 | H | $C_2H_5$ | $C_2H_5$ | $CH_3$ | |
| 9.030 | Cl | 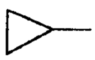 | $CH_3$ | $CH_3$ | $CH_3$ | H | |
| 9.031 | Cl | 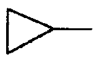 | $CH_3$ | $C_2H_5$ | $C_2H_5$ | H | |
| 9.032 | Cl | $nC_3H_7$ | H | $CH_3$ | $CH_3$ | H | |
| 9.033 | Cl | $nC_3H_7$ | H | $C_2H_5$ | $C_2H_5$ | H | |
| 9.034 | Cl | $CH_3$ | H | $-CH_2CH_2CH_2CH_2-$ | | H | |
| 9.035 | Cl | $CH_3$ | H | $-CH_2CH_2CH_2CH_2CH_2-$ | | H | |
| 9.036 | Cl | H | $CH_3$ | $CH_3$ | $CH_3$ | H | |
| 9.037 | Cl | H | $CH_3$ | $CH_3$ | $CH_3$ | $CH_3$ | |
| 9.038 | H | $CH_3$ | H | $CH_3$ | $CH_3$ | H | $n_D^{23} = 1{,}5859$ |
| 9.039 | H | $CH_3$ | H | $CH_3$ | $CH_3$ | $CH_3$ | |
| 9.040 | H | $CH_3$ | H | $C_2H_5$ | $C_2H_5$ | H | |
| 9.041 | H | H | H | $CH_3$ | $CH_3$ | H | |
| 9.042 | H | $C_2H_5$ | H | $CH_3$ | $CH_3$ | H | |
| 9.043 | H | 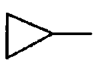 H | | $CH_3$ | $CH_3$ | H | |
| 9.044 | Cl | $CH_3$ | H | $CH_3$ | $CH_3$ | 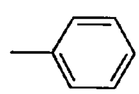 | |
| 9.045 | Cl | $CH_3$ | H | $CH_3$ | $CH_3$ | $-CH_2OCH_3$ | |
| 9.046 | Cl | $CH_3$ | H | $CH_3$ | $CH_3$ | -CN | |

Tabelle 9 (Fortsetzung)

| Verb. Nr. | Y | $R_1$ | $R_2$ | $R_3$ | $R_4$ | $R_5$ | Schmp. |
|---|---|---|---|---|---|---|---|
| 9.047 | Cl | $CH_3$ | H | $CH_3$ | $CH_3$ | $-COOC_2H_5$ | |
| 9.048 | Cl | $CH_3$ | H | $CH_3$ | $CH_3$ | $-CON(CH_3)_2$ | |
| 9.049 | Cl | $CH_3$ | H | $CH_3$ | $-CH_2CH_2COOC_2H_5-$ | H | |

Tabelle 10

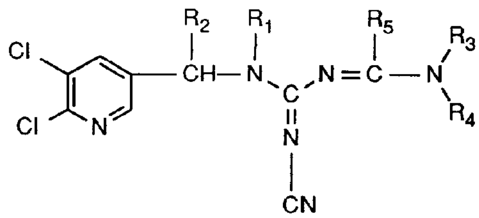

| Verb. Nr. | $R_1$ | $R_2$ | $R_3$ | $R_4$ | $R_5$ | Schmp. |
|---|---|---|---|---|---|---|
| 10.001 | $CH_3$ | H | $CH_3$ | $CH_3$ | H | 117-120°C |
| 10.002 | H | H | $CH_3$ | $CH_3$ | H | |
| 10.003 | $CH_3$ | H | $CH_3$ | $CH_3$ | $CH_3$ | $n_D^{23}$= 1,5802 |
| 10.004 | $CH_3$ | H | $CH_3$ | $C_2H_5$ | H | |
| 10.005 | $CH_3$ | H | $C_2H_5$ | $C_2H_5$ | H | |
| 10.006 | $CH_2$ | H | $nC_3H_7$ | $nC_3H_7$ | H | |
| 10.007 | $CH_3$ | H | $C_2H_5$ | $CH_3$ | $CH_3$ | |
| 10.008 | H | H | $CH_3$ | $CH_3$ | $CH_3$ | |
| 10.009 | $CH_3$ | H | $C_2H_5$ | $C_2H_5$ | $CH_3$ | |
| 10.010 | $CH_3$ | $CH_3$ | $CH_3$ | $CH_3$ | H | |
| 10.011 | $CH_3$ | $CH_3$ | $C_2H_5$ | $C_2H_5$ | H | |
| 10.012 | $C_2H_5$ | H | $CH_3$ | $CH_3$ | H | 102-103°C |
| 10.013 | $C_2H_5$ | H | $CH_3$ | $CH_3$ | $CH_3$ | |
| 10.014 | $C_2H_5$ | H | $CH_3$ | $C_2H_5$ | H | |
| 10.015 | $C_2H_5$ | H | $C_2H_5$ | $C_2H_5$ | H | |
| 10.016 | $C_2H_5$ | H | $nC_3H_7$ | $nC_3H_7$ | H | |
| 10.017 | $C_2H_5$ | H | $CH_3$ | $CH_3$ | $C_2H_5$ | |
| 10.018 | $C_2H_5$ | H | $C_2H_5$ | $CH_3$ | $CH_3$ | |
| 10.019 | $C_2H_5$ | H | $C_2H_5$ | $C_2H_5$ | $CH_3$ | |
| 10.020 | $C_2H_5$ | $CH_3$ | $CH_3$ | $CH_3$ | H | |
| 10.021 | $C_2H_5$ | $CH_3$ | $C_2H_5$ | $C_2H_5$ | H | |
| 10.022 | 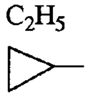 | H | $CH_3$ | $CH_3$ | H | |
| 10.023 | 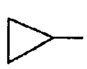 | H | $CH_3$ | $CH_3$ | $CH_3$ | |

Tabelle 10 (Fortsetzung)

| Verb. Nr. | $R_1$ | $R_2$ | $R_3$ | $R_4$ | $R_5$ | Schmp. |
|---|---|---|---|---|---|---|
| 10.024 | 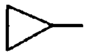 | H | $CH_3$ | $C_2H_5$ | H | |
| 10.025 | 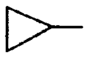 | H | $C_2H_5$ | $C_2H_5$ | H | |
| 10.026 | 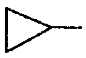 | H | $nC_3H_7$ | $nC_3H_7$ | H | |
| 10.027 | 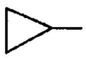 | H | $CH_3$ | $CH_3$ | $C_2H_5$ | |
| 10.028 | 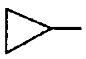 | H | $C_2H_5$ | $CH_3$ | $CH_3$ | |
| 10.029 | 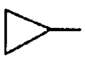 | H | $C_2H_5$ | $C_2H_5$ | $CH_3$ | |
| 10.030 | 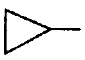 | $CH_3$ | $CH_3$ | $CH_3$ | H | |
| 10.031 | 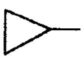 | $CH_3$ | $C_2H_5$ | $C_2H_5$ | H | |
| 10.032 | $nC_3H_7$ | H | $CH_3$ | $CH_3$ | H | |
| 10.033 | $nC_3H_7$ | H | $C_2H_5$ | $C_2H_5$ | H | |
| 10.034 | $CH_3$ | H | $-CH_2CH_2CH_2CH_2-$ | | H | |
| 10.035 | $CH_3$ | H | $-CH_2CH_2CH_2CH_2CH_2-$ | | H | |
| 10.036 | H | $CH_3$ | $CH_3$ | $CH_3$ | H | |
| 10.037 | H | $CH_3$ | $CH_3$ | $CH_3$ | $CH_3$ | |
| 10.038 | $CH_3$ | H | $CH_3$ | $CH_3$ | 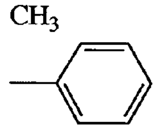 | |
| 10.039 | $CH_3$ | H | $CH_3$ | $CH_3$ | $-CH_2OCH_3$ | |
| 10.040 | $CH_3$ | H | $CH_3$ | $CH_3$ | -CN | |
| 10.041 | $CH_3$ | H | $CH_3$ | $CH_3$ | $-COOC_2H_5$ | |
| 10.042 | $CH_3$ | H | $CH_3$ | $CH_3$ | $-CON(CH_3)_2$ | |
| 10.043 | $CH_3$ | H | $CH_3$ | $CH_2CH_2COOC_2H_5$ | H | |

Tabelle 11

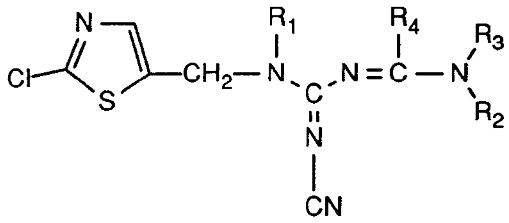

| Verb. Nr. | $R_1$ | $R_2$ | $R_3$ | $R_4$ | Schmp. |
|---|---|---|---|---|---|
| 11.001 | $CH_3$ | $CH_3$ | $CH_3$ | H | |
| 11.002 | H | $CH_3$ | $CH_3$ | H | |
| 11.003 | $CH_3$ | $CH_3$ | $CH_3$ | $CH_3$ | |
| 11.004 | $CH_3$ | $CH_3$ | $C_2H_5$ | H | |
| 11.005 | $CH_3$ | $C_2H_5$ | $C_2H_5$ | H | |
| 11.006 | $CH_3$ | $C_2H_5$ | $CH_3$ | $CH_3$ | |
| 11.007 | $CH_3$ | $C_2H_5$ | $C_2H_5$ | $CH_3$ | |
| 11.008 | $C_2H_5$ | $CH_3$ | $CH_3$ | H | |
| 11.009 | $C_2H_5$ | $CH_3$ | $CH_3$ | $CH_3$ | |
| 11.010 | $C_2H_5$ | $CH_3$ | $C_2H_5$ | H | |
| 11.011 | $C_2H_5$ | $C_2H_5$ | $C_2H_5$ | H | |
| 11.012 | $C_2H_5$ | $nC_3H_7$ | $nC_3H_7$ | H | |
| 11.013 | $C_2H_5$ | $CH_3$ | $CH_3$ | $C_2H_5$ | |
| 11.014 | $C_2H_5$ | $C_2H_5$ | $CH_3$ | $CH_3$ | |
| 11.015 | $C_2H_5$ | $C_2H_5$ | $C_2H_5$ | $CH_3$ | |
| 11.016 | 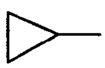 | $CH_3$ | $CH_3$ | H | |
| 11.017 | 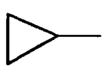 | $CH_3$ | $CH_3$ | $CH_3$ | |
| 11.018 | 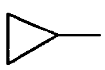 | $CH_3$ | $C_2H_5$ | H | |
| 11.019 | 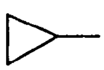 | $C_2H_5$ | $C_2H_5$ | H | |
| 11.020 | 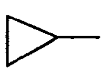 | $nC_3H_7$ | $nC_3H_7$ | H | |

Tabelle 11 (Fortsetzung)

| Verb. Nr. | $R_1$ | $R_2$ | $R_3$ | $R_4$ | Schmp. |
|---|---|---|---|---|---|
| 11.021 | 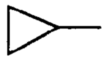 | $C_2H_5$ | $CH_3$ | $CH_3$ | |
| 11.022 | 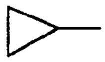 | $C_2H_5$ | $C_2H_5$ | $CH_3$ | |
| 11.023 | $nC_3H_7$ | $CH_3$ | $Ch_3$ | H | |
| 11.024 | $nC_3H_7$ | $C_2H_5$ | $C_2H_5$ | H | |
| 11.025 | $CH_3$ | $-CH_2CH_2CH_2CH_2-$ | | H | |
| 11.026 | $CH_3$ | $-CH_2CH_2CH_2CH_2CH_2-$ | | H | |
| 11.027 | H | $CH_3$ | $CH_3$ | $CH_3$ | |
| 11.028 | $CH_3$ | $CH_3$ | $CH_3$ | 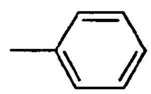 | |
| 11.029 | $CH_3$ | $CH_3$ | $CH_3$ | $-CH_2OCH_3$ | |
| 11.030 | $CH_3$ | $CH_3$ | $CH_3$ | -CN | |
| 11.031 | $CH_3$ | $CH_3$ | $CH_3$ | $-COOC_2H_5$ | |
| 11.032 | $CH_3$ | $CH_3$ | $CH_3$ | $-CON(CH_3)_2$ | |
| 11.033 | $CH_3$ | $CH_3$ | $-CH_2CH_2COOC_2H_5-$ | H | |

Tabelle 12

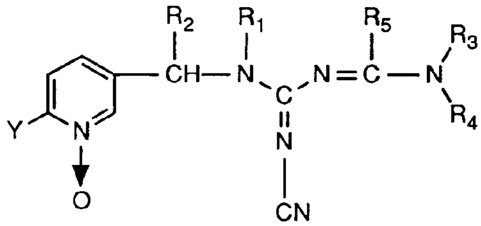

| Verb. Nr. | Y | $R_1$ | $R_2$ | $R_3$ | $R_4$ | $R_5$ | Schmp. |
|---|---|---|---|---|---|---|---|
| 12.001 | Cl | $CH_3$ | H | $CH_3$ | $CH_3$ | H | |
| 12.002 | Cl | H | H | $CH_3$ | $CH_3$ | H | |
| 12.003 | Cl | $CH_3$ | H | $CH_3$ | $CH_3$ | $CH_3$ | |
| 12.004 | Cl | $CH_3$ | H | $C_2H_5$ | $C_2H_5$ | H | |
| 12.005 | Cl | H | H | $CH_3$ | $CH_3$ | $CH_3$ | |
| 12.006 | Cl | $CH_3$ | H | $C_2H_5$ | $C_2H_5$ | $CH_3$ | |
| 12.007 | Cl | $CH_3$ | $CH_3$ | $CH_3$ | $CH_3$ | H | |
| 12.008 | Cl | $C_2H_5$ | H | $CH_3$ | $CH_3$ | H | |
| 12.009 | Cl | $C_2H_5$ | H | $CH_3$ | $CH_3$ | $CH_3$ | |
| 12.010 | Cl | $C_2H_5$ | H | $C_2H_5$ | $C_2H_5$ | H | |
| 12.011 | Cl | $C_2H_5$ | $CH_3$ | $CH_3$ | $CH_3$ | H | |
| 12.012 | Cl | 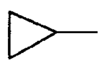 | H | $CH_3$ | $CH_3$ | H | |
| 12.013 | Cl | 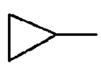 | H | $CH_3$ | $CH_3$ | $CH_3$ | |
| 12.014 | Cl | 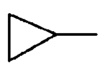 | H | $C_2H_5$ | $C_2H_5$ | H | |
| 12.015 | Cl | 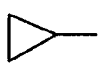 | $CH_3$ | $CH_3$ | $CH_3$ | H | |
| 12.016 | H | $CH_3$ | H | $CH_3$ | $CH_3$ | H | |
| 12.017 | H | $CH_3$ | H | $CH_3$ | $CH_3$ | $CH_3$ | |
| 12.018 | H | $CH_3$ | H | $C_2H_5$ | $C_2H_5$ | H | |
| 12.019 | H | H | H | $CH_3$ | $CH_3$ | H | |
| 12.020 | H | $C_2H_5$ | H | $CH_3$ | $CH_3$ | H | |
| 12.021 | H | 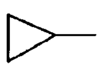 | H | $CH_3$ | $CH_3$ | H | |

Tabelle 12 (Fortsetzung)

| Verb. Nr. | Y | $R_1$ | $R_2$ | $R_3$ | $R_4$ | $R_5$ | Schmp. |
|---|---|---|---|---|---|---|---|
| 12.022 | Cl | $CH_3$ | H | $CH_3$ | $CH_3$ | 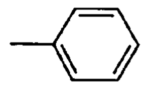 | |
| 12.023 | Cl | $CH_3$ | H | $CH_3$ | $CH_3$ | $-CH_2OCH_3$ | |
| 12.024 | Cl | $CH_3$ | H | $CH_3$ | $CH_3$ | -CN | |
| 12.025 | Cl | $CH_3$ | H | $CH_3$ | $CH_3$ | $-COOC_2H_5$ | |
| 12.026 | Cl | $CH_3$ | H | $CH_3$ | $CH_3$ | $-CON(CH_3)_2$ | |
| 12.027 | Cl | $CH_3$ | H | $CH_3$ | $CH_2CH_2COOC_2H_5$ | H | |

Tabelle 13

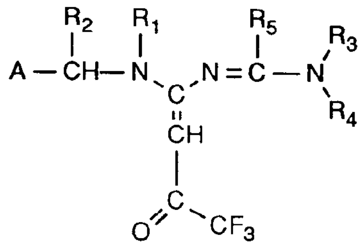

| Verb. Nr. | A | $R_1$ | $R_2$ | $R_3$ | $R_4$ | $R_5$ | Schmp. |
|---|---|---|---|---|---|---|---|
| 13.001 | 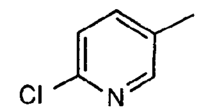 | $CH_3$ | H | $CH_3$ | $CH_3$ | H | |
| 13.002 | 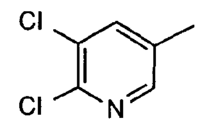 | $CH_3$ | H | $CH_3$ | $CH_3$ | H | |
| 13.003 | 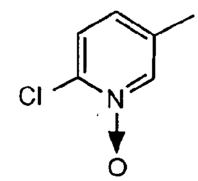 | $CH_3$ | H | $CH_3$ | $CH_3$ | H | |
| 13.004 | 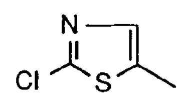 | $CH_3$ | H | $CH_3$ | $CH_3$ | H | |

Formulierungsbeispiele (% = Gewichtsprozent)

Beispiel F1: Emulsions-Konzentrate

| | a) | b) | c) |
|---|---|---|---|
| Wirkstoff Nr. 1.003 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 9.038 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 9.003 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 9.003 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr. 1.001, 1.002, 1.003, 1.038 oder 9.001 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel F6: Emulsions-Konzentrat

| | |
|---|---|
| Wirkstoff Nr. 1.001, 1.002, 1.003, 1.038 oder 9.001 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 1.001, 1.002, 1.003, 1.038 oder 9.001 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff Nr. 1.001, 1.002, 1.003, 1.038 oder 9.001 | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff Nr. 1.001, 1.002 1.003, 1.038 oder 9.001 | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

Beispiel F10: Suspensions-Konzentrat

| | |
|---|---|
| Wirkstoff Nr. 1.001, 1.002, 1.003, 1.038 oder 9.001 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff oder die Wirkstoffkombination wird mit den Zusatzstoffen innig vermischt. Man erhält so ein Suspensions-konzentrat, aus welchem durch Verdünnen mit Wasser Suspensionen jeder gewünschten Konzentration hergestellt werden können.

Biologische Beispiele

Beispiel B1: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3.

Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt. Verbindungen der Tabellen 1 bis 13 zeigen eine gute Wirkung gegen Nilaparvata lugens in diesem Test. Insbesondere die Verbindungen 1.001, 1.022, 1.038, 5.001 und 9.001 zeigen eine Wirkung über 80 %.

Beispiel B2: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt. Verbindungen der Tabellen 1 bis 13 zeigen eine gute Wirkung gegen Nephotettix cincticeps in diesem Test. Insbesondere die Verbindungen 1.001, 1.022 und 5.001 zeigen eine Wirkung über 80 %.

Beispiel B3: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden die Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen. Verbindungen gemäss Tabellen 1 bis 13 zeigen in diesem Test gute Wirkung gegen Bemisia tabaci. Insbesondere die Verbindungen 1.001 und 5.001 zeigen eine Wirkung über 80 %.

Beispiel B4: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt. Verbindungen der Tabellen 1 bis 13 zeigen eine gute Wirkung gegen Aphis craccivora in diesem Test. Insbesondere die Verbindungen 1.001, 1.003, 1.012, 1.022, 1.038, 5.001 und 9.00 1 zeigen eine Wirkung über 80 %.

Beispiel B5: Wirkung gegen Myzus persicae

Erbsenkeimlinge werden mit Myzus persicae infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pfanzen wird die prozentuale Reduktion der Population (% Wirkung) bestimmt. Verbindungen der Tabellen 1 bis 13 zeigen eine gute Verbindung gegen Myzus persicae in diesem Test. Insbesondere die Verbindungen 1.001 und 1.022 zeigen eine Wirkung über 80 %.

Beispiel B6: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt. Verbindungen der Tabellen 1 bis 13 zeigen eine gute Wirkung gegen Myzus persicae in diesem Test. Insbesondere die Verbindungen 1.001, 1.003, 1.012, 1.022, 1.038 und 5.001 zeigen eine Wirkung über 80 %.

Beispiel B7: Systemische Wirkung gegen Nilaparvata lugens

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den

unbehandelten Pflanzen wird die prozentuale Reduktion der Population (% Wirkung) bestimmt. Verbindungen der Tabellen 1 bis 13 zeigen eine gute Wirkung gegen Nilaparvata lugens in diesem Test. Insbesondere die Verbindungen 1.001, 1.012, 1.022, 1.038, 3.001, 5.001 und 9.001 zeigen eine Wirkung über 80 %.

Beispiel B8: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.
Verbindungen der Tabellen 1 bis 13 zeigen eine gute Wirkung gegen Nephotettix cincticeps in diesem Test. Insbesondere die Verbindungen 1.001, 1.012, 1.022, 3.001, 5.001 und 9.001 zeigen eine Wirkung über 80 %.

Beispiel B9: Frasswirkung gegen Ctenocephalides felis (systemisch)

20 adulte Flöhe der Art Ctenocephalides felis werden in einen flachen runden Käfig gegeben, der auf beiden Seiten mit Gaze verschlossen ist. Auf diesen Käfig wird nun ein Gefäss gestellt, das auf der unteren Seite mit einer Parafilmmembran verschlossen ist. Das Gefäss enthält Blut, das 50 ppm des Wirkstoffs enthält und konstant auf 37°C erwärmt wird. Die Flöhe nehmen das Blut durch die Membran auf. 24 und 48 Stunden nach Ansatz erfolgt die Auswertung. Aus dem Vergleich der Anzahl toter Flöhe mit behandeltem Blut zu denjenigen mit unbehandeltem Blut wird die prozentuale Reduktion der Population (% Wirkung) bestimmt. 24 Stunden nach Behandlung wird das Blut durch neues ebenfalls behandeltes ersetzt.
Die Verbindungen der Tabellen 1 bis 13 zeigen eine gute Wirkung gegen Ctenocephalides felis in diesem Test. Insbesondere zeigen die Verbindungen 1.001 und 5.001 eine Wirkung über 80 %.

## Patentansprüche

1. Eine Verbindung der Formel

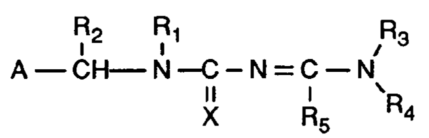

(I),

worin
$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl und
$R_4$ für $C_1$-$C_4$-Alkyl, $C_3$-$C_6$-Cycloalkyl oder $-CH_2CH_2COOR_7$ oder
$R_3$ und $R_4$ gemeinsam für $-(CH_2)_4-$ oder $-(CH_2)_5-$,
$R_5$ für Wasserstoff, $C_1$-$C_4$-Alkyl,

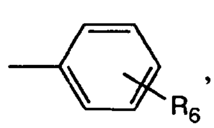

,

$-CH_2OCH_3$, -CN, $-COOR_7$ oder

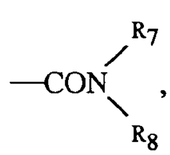

,

$R_6$ für Wasserstoff, Chlor, Methyl oder Nitro,

$R_7$ und $R_8$ für Methyl oder Ethyl,
A für einen unsubstituierten oder ein- bis vierfach substituierten, aromatischen oder nicht-aromatischen, monocyclischen oder bicyclischen, heterocyclischen Rest, wobei ein bis zwei Substituenten aus der Gruppe, bestehend aus $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyano und Nitro, und ein bis vier Substituenten aus der Gruppe, bestehend aus $C_1$-$C_3$-Alkyl, $C_1$-$C_3$-Alkoxy und Halogen, ausgewählt sind, und =X für =N-$NO_2$, =CH-$NO_2$, =N-CN oder =CH-CO-$CF_3$ stehen,
in freier Form oder in Säureadditionssalzform.

2. Eine Verbindung gemäss Anspruch 1 der Formel I in freier Form.

3. Eine Verbindung gemäss Anspruch 2 der Formel I, worin der Rest A ungesättigt und über ein Kohlenstoff-Ringglied an das den Rest $R_2$ tragende Kohlenstoffatom der Formel I gebunden ist.

4. Eine Verbindung gemäss Anspruch 3 der Formel I, worin der Rest A aromatischen Charakter aufweist.

5. Eine Verbindung gemäss Anspruch 4 der Formel I, worin der Rest A mindestens ein Stickstoffatom enthält.

6. Eine Verbindung gemäss Anspruch 4 der Formel I, worin der Rest A ein bis drei Heteroatome aus der Gruppe, bestehend aus Sauerstoff, Schwefel und Stickstoff, enthält, wobei jeweils höchstens eines der Heteroatome ein Sauerstoff- oder ein Schwefelatom ist.

7. Eine Verbindung gemäss Anspruch 5 der Formel I, worin der Rest A ein bis drei Heteroatome aus der Gruppe, bestehend aus Sauerstoff, Schwefel und Stickstoff, enthält, wobei stets mindestens ein Stickstoffatom in A enthalten ist und höchstens eines der gegebenenfalls weiteren in A enthaltenen Heteroatome ein Sauerstoffatom oder ein Schwefelatom ist

8. Eine Verbindung gemäss Anspruch 4 der Formel I, worin der Rest A ausgewählt ist aus der Gruppe, bestehend aus den Grundkörpern

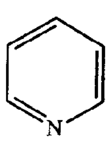 , 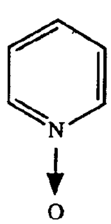 , 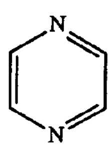 , 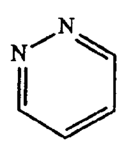 , 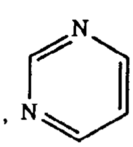 , 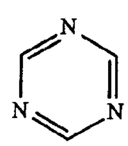 , 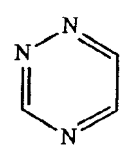 ,

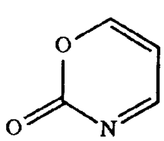 , 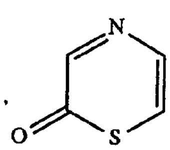 , 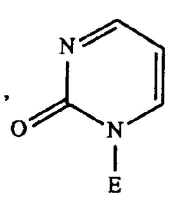 , 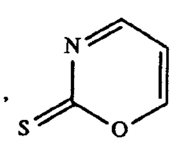 , 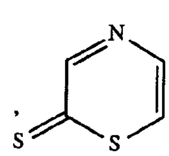 , 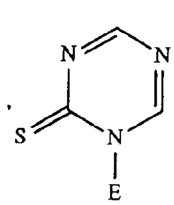 ,

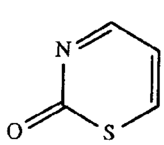 , 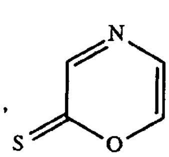 , 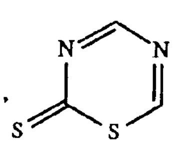 , , 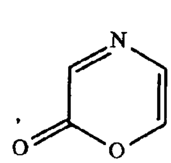 .

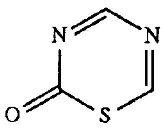 , 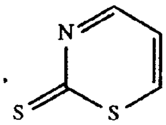 , 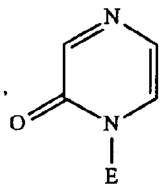 , 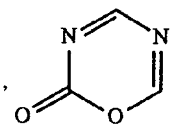 , 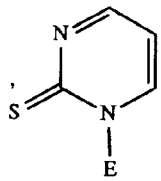 , 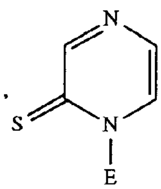 ,

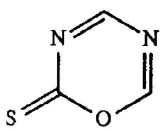 , 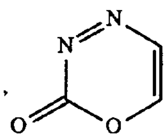 , 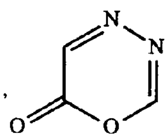 , 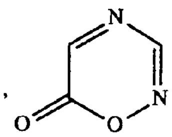 , 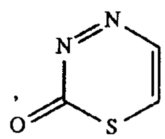 ,

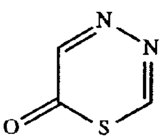 , 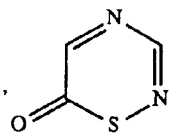 , 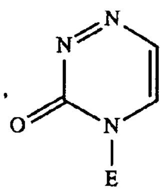 , 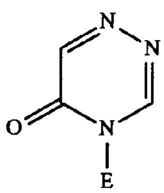 , 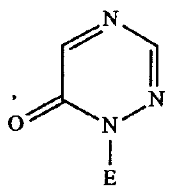 ,

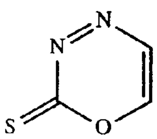 , 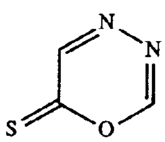 , 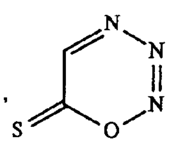 , 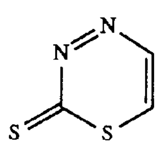 , 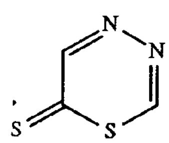 ,

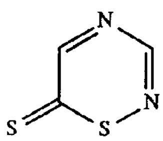 , 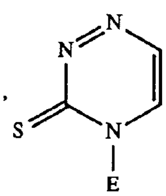 , 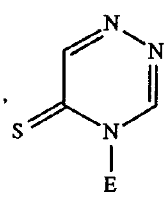 , 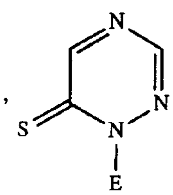 , 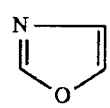 ,

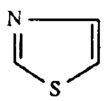 , 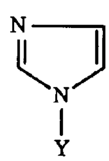 , 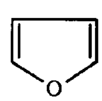 , 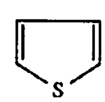 ,

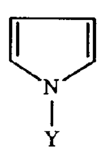 , 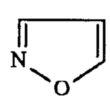 , 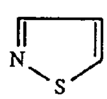 , 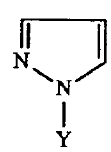 ,

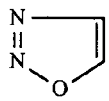 , 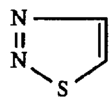 , 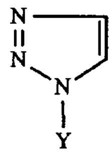 , 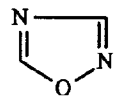 , 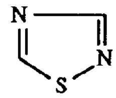 ,

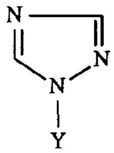 , 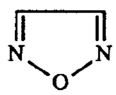 , 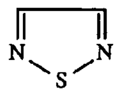 , 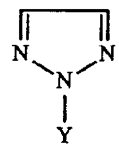 , 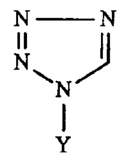 ,

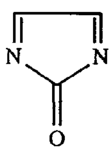 , 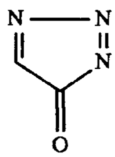 , 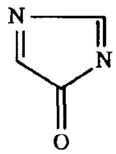 , 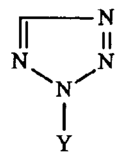 , 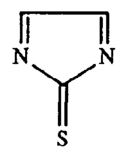 ,

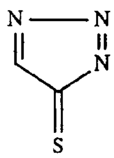 , 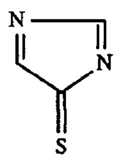 , 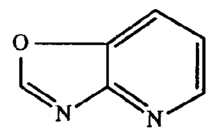 , 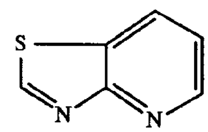 , 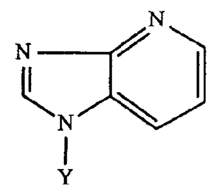 ,

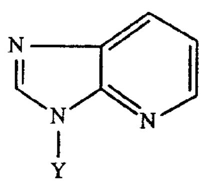 , 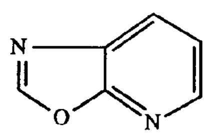 , 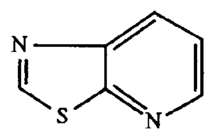 , 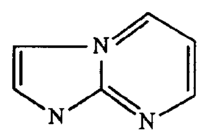 ,

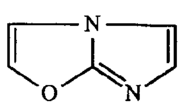 , 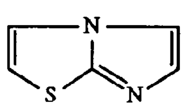 , 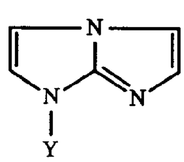 ,

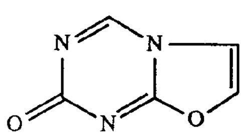 , 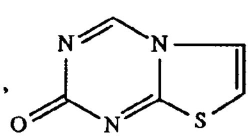 , 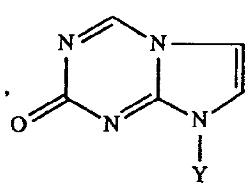 , 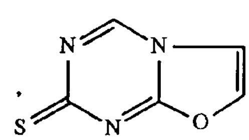 ,

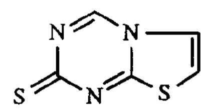

und

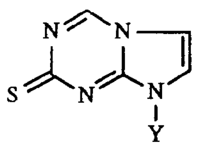,

welche unsubstituiert sind oder je nach den Substitutionsmöglichkeiten des Ringsystems bis zu vier der in Anspruch 1 definierten Substituenten tragen, worin E für $C_1$-$C_3$-Alkyl und Y für Wasserstoff, $C_1$-$C_3$-Alkyl oder Cyclopropyl stehen und welche über ein Kohlenstoffatom des Heterocyclus an das den Rest $R_2$ tragende Kohlenstoffatom der Formel I gebunden sind.

**9.** Eine Verbindung gemäss Anspruch 8 der Formel I, worin der Rest A unsubstituiert ist oder einen oder zwei Substituenten, ausgewählt aus der Gruppe, bestehend aus Halogen, $C_1$-$C_3$-Alkyl, $C_1$-$C_3$-Halogenalkyl, $C_1$-$C_3$-Halogenalkoxy und $C_1$-$C_3$-Alkoxy, trägt.

**10.** Eine Verbindung gemäss Anspruch 9 der Formel I, worin der Rest A ein von Pyridin oder Thiazol abgeleiteter Rest ist.

**11.** Eine Verbindung gemäss Anspruch 10 der Formel I, worin der Rest A Pyrid-3-yl, 2-Halogenpyrid-5-yl, 2,3-Dihalogenpyrid-5-yl, 2-Halogen-1-oxido-5-pyridinio oder 2-Halogenthiazol-5-yl ist.

**12.** Eine Verbindung gemäss Anspruch 11 der Formel I, worin =X für $=N-NO_2$, =N-CN oder $=CH-NO_2$ steht.

**13.** Eine Verbindung gemäss Anspruch 12 der Formel I, worin
$R_1$ für Wasserstoff, $C_1$-$C_3$-Alkyl oder Cyclopropyl,
$R_2$ für Wasserstoff oder Methyl,
$R_3$ für $C_1$-$C_3$-Alkyl und
$R_4$ für $C_1$-$C_3$-Alkyl oder Ethoxycarbonylethyl oder
$R_3$ und $R_4$ gemeinsam für $-(CH_2)4-$ oder $-(CH_2)_5-$ und
$R_5$ für Wasserstoff, Methyl, Ethyl, Phenyl $-CH_2OCH_3$, -CN, $-COOC_2H_5$ oder

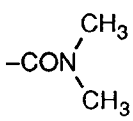

stehen.

**14.** Eine Verbindung gemäss Anspruch 13 der Formel I, worin
$R_1$ für Methyl, Ethyl oder Cyclopropyl,
$R_2$ für Wasserstoff,
$R_3$ für Methyl,
$R_4$ für Methyl und
$R_5$ für Wasserstoff oder Methyl stehen.

**15.** Eine Verbindung gemäss Anspruch 14 der Formel I, worin der Rest A 2-Chlorpyrid-5-yl ist.

**16.** Eine Verbindung gemäss Anspruch 15 der Formel I, worin =X für $=N-NO_2$ oder $=CH-NO_2$ steht.

**17.** N-(2-Chlorpyrid-5-ylmethyl)-N-methyl-N′-(N,N-dimethylaminomethylen)-N″-nitro-guanidin gemäss Anspruch 1.

**18.** 1-[N-(2-Chlorpyrid-5-ylmethyl)-N-methyl-amino]-1-(N,N-dimethylaminomethylenamino)-2-nitro-äthen gemäss Anspruch 1.

**19.** N-(2-Chlorpyrid-5-ylmethyl)-N-cyclopropyl-N′-(N,N-dimethylaminomethylen)-N″-nitro-guanidin gemäss Anspruch 1.

**20.** Eine Verbindung gemäss Anspruch 1 der Formel I, ausgewählt aus der Gruppe, bestehend aus den Verbindungen

(b) N-(2-Chlorpyrid-5-ylmethyl)-N-methyl-N'-[1-(N,N-dimethylamino)ethyliden]-N"-nitro-guanidin,
(c) N-Pyrid-3-ylmethyl-N-methyl-N'-(N,N-dimethylaminomethylen)-N"-nitro-guanidin,
(d) N-(2-Chlorpyrid-5-ylmethyl)-N-methyl-N'-(N,N-dimethylaminomethylen)-N"-nitro-guanidin,
(f) 1-[N-(2-Chlorpyrid-5-ylmethyl)-N-ethyl-amino]-1-(N,N-dimethylaminomethylenamino)-2-nitro-äthen,
(g) 1-[N-(2-Chlorpyrid-5-ylmethyl)-N-cyclopropyl-amino]-1-(N,N-dimethylaminomethylenamino)-2-nitro-äthen,
(h) 1-[N-(2-Chlorthiazol-5-ylmethyl)-N-methyl-amino]-1-(N,N-dimethylaminomethylenamino)-2-nitro-äthen,
(i) 1-[N-(2-Chlorthiazol-5-ylmethyl)-N-ethyl-amino]-1-(N,N-dimethylaminomethylenamino)-2-nitro-äthen,
(j)) 1-[N-(2-Chlorthiazol-5-ylmethyl)-N-cyclopropyl-amino]-1-(N,N-dimethylaminomethylenamino)-2-nitro-äthen,
(k) 1-[N-(2-Chlor-1-oxido-5-pyridiniomethyl)-N-methyl-amino]-1-(N,N-dimethylaminomethylenamino)-2-nitro-äthen,
(l) 1-[N-(2-Chlor-1-oxido-5-pyridiniomethyl)-N-ethyl-amino]-1-(N,N-dimethylaminomethylenamino)-2-nitro-äthen,
(m) 1-[N-(2-Chlor-1-oxido-5-pyridiniomethyl)-N-cyclopropyl-amino]-1-(N,N-dimethylaminomethylenamino)-2-nitro-äthen,
(n) N-(2-Chlorthiazol-5-ylmethyl)-N-methyl-N'-(N,N-dimethylaminomethylen)-N"-nitro-guanidin,
(o) N-(2-Chlorthiazol-5-ylmethyl)-N-ethyl-N'-(N,N-dimethylaminomethylen)-N"-nitro-guanidin,
(p) N-(2-Chlorthiazol-5-ylmethyl)-N-cyclopropyl-N'-(N,N-dimethylaminomethylen)-N"-nitro-guanidin und
(q) N-(2-Chlorpyrid-5-ylmethyl)-N-ethyl-N'-(N,N-dimethylaminomethylen)-N"-nitro-guanidin.

**21.** Verfahren zur Herstellung einer Verbindung gemäss Anspruch 1 der Formel I, in freier Form oder in Säureadditionssalzform, dadurch gekennzeichnet, dass man

a) eine Verbindung der Formel

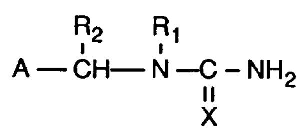

(II),

worin $R_1$, $R_2$, A und X die für die Formel I angegebenen Bedeutungen haben, in einem inerten aprotischen Lösungsmittel mit einer Verbindung der Formel

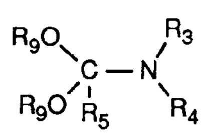

(III),

worin $R_3$, $R_4$ und $R_5$ die für die Formel I angegebenen Bedeutungen haben und $R_9$ $C_1$-$C_4$-Alkyl ist, umsetzt oder

b) eine Verbindung der Formel

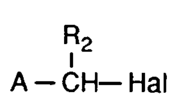

(IV),

worin $R_2$ und A die für die Formel I angegebenen Bedeutungen haben und Hal für Chlor, Brom oder Iod steht, in Gegenwart eines Säureakzeptors in einem Lösungsmittel bei -20 bis +120°C mit einer Verbindung der Formel

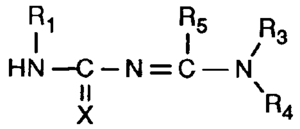

(V),

worin $R_1$, $R_3$, $R_4$, $R_5$ und X die für die Formel I angegebenen Bedeutungen haben, umsetzt und jeweils die entstehende Verbindung der Formel I isoliert oder in ein Säureadditionssalz überführt und dieses isoliert.

**22.** Schädlingsbekämpfungsmittel, dadurch gekennzeichnet, dass es mindestens eine Verbindung gemäss Anspruch 1 der Formel I, in freier Form oder in agrochemisch verwendbarer Säureadditionssalzform, als Wirkstoff und mindestens einen Hilfsstoff enthält.

**23.** Mittel gemäss Anspruch 22 zur Bekämpfung von Insekten und/oder Spinnentieren.

**24.** Verfahren zur Herstellung eines Mittels gemäss Anspruch 22 oder 23, dadurch gekennzeichnet, dass man den Wirkstoff mit dem (den) Hilfsstoff(en) innig vermischt.

**25.** Verwendung einer Verbindung gemäss Anspruch 1 der Formel I, in freier Form oder in agrochemisch verwendbarer Säureadditionssalzform, oder eines Mittels gemäss Anspruch 22 zur Bekämpfung von Schädlingen.

**26.** Verwendung gemäss Anspruch 25 zur Bekämpfung von Insekten und/oder Spinnentieren.

**27.** Verwendung gemäss Anspruch 25 zur Behandlung von Saatgut.

**28.** Verwendung einer Verbindung gemäss Anspruch 1 der Formel I, in freier Form oder in agrochemisch verwendbarer Säureadditionssalzform, zur Herstellung eines Mittels gemäss Anspruch 22 oder 23.

**29.** Verfahren zur Bekämpfung von Schädlingen, dadurch gekennzeichnet, dass man eine Verbindung gemäss Anspruch 1 der Formel I, in freier Form oder in agrochemisch verwendbarer Säureadditionssalzform, oder ein Mittel gemäss Anspruch 22 oder 23 auf die Schädlinge und/oder ihren Lebensraum appliziert.

**30.** Verfahren gemäss Anspruch 29 zur Bekämpfung von Insekten und/oder Spinnentieren.

**31.** Verfahren gemäss Anspruch 29 zum Schutz von Saatgut, dadurch gekennzeichnet, dass man das Saatgut oder die Saatfurche behandelt.

**32.** Saatgut, behandelt gemäss dem in Anspruch 31 beschriebenen Verfahren.

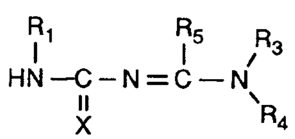

(V),

worin $R_1$, $R_3$, $R_4$, $R_5$ und X die in Anspruch 1 für die Formel I angegebenen Bedeutungen haben.

**33.** Eine Verbindung gemäss Anspruch 33 der Formel V, ausgewählt aus der Gruppe, bestehend aus den Verbindungen
N-Cyclopropyl-N′-(N,N-dimethylaminomethylen)-N″-nitro-guanidin,
N-(N,N-Dimethylaminomethylen)-N′-methyl-N″-nitro-guanidin und
1-(N,N-Dimethylaminomethylenamino)-1-(N-methylamino)-2-nitro-aethen.

**Patentansprüche für den benannten Vertragsstaat ES:**

**1.** Verfahren zur Herstellung einer Verbindung der Formel

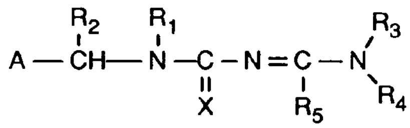 (I),

worin
$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl und
$R_4$ für $C_1$-$C_4$-Alkyl, $C_3$-$C_6$-Cycloalkyl oder $-CH_2CH_2COOR$, oder
$R_3$ und $R_4$ gemeinsam für $-(CH_2)_4-$ oder $-(CH_2)_5-$,
$R_5$ für Wasserstoff, $C_1$-$C_4$-Alkyl,

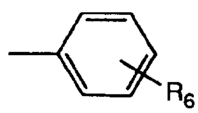 ,

$-CH_2OCH_3$, -CN, -COOR, oder

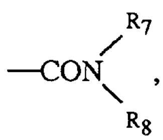 ,

$R_6$ für Wasserstoff, Chlor, Methyl oder Nitro,
$R_7$ und $R_8$ für Methyl oder Ethyl,
A für einen unsubstituierten oder ein- bis vierfach substituierten, aromarischen oder nicht-aromatischen, monocyclischen oder bicyclischen, heterocyclischen Rest, wobei ein bis zwei Substituenten aus der Gruppe, bestehend aus $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyano und Nitro, und ein bis vier Substituenten aus der Gruppe, bestehend aus $C_1$-$C_3$-Alkyl, $C_1$-$C_3$-Alkoxy und Halogen, ausgewählt sind, und =X für $=N-NO_2$, $=CH-NO_2$, =N-CN oder $=CH-CO-CF_3$ stehen,
in freier Form oder in Säureadditionssalzform,
dadurch gekennzeichnet, dass man

a) eine Verbindung der Formel

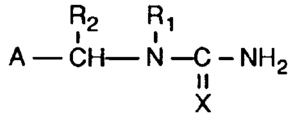 (II),

worin $R_1$, $R_2$, A und X die für die Formel I angegebenen Bedeutungen haben, in einem inerten aprotischen Lösungsmittel mit einer Verbindung der Formel

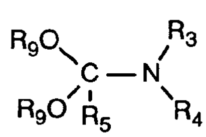 (III),

worin $R_3$, $R_4$ und $R_5$ die für die Formel I angegebenen Bedeutungen haben und $R_9$ $C_1$-$C_4$-Alkyl ist, umsetzt oder

b) eine Verbindung der Formel

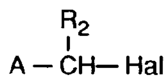

(IV),

worin $R_2$ und A die für die Formel I angegebenen Bedeutungen haben und Hal für Chlor, Brom oder Iod steh, in Gegenwart eines Säureakzeptors in einem Lösungsmittel bei -20 bis +120°C mit einer Verbindung der Formel

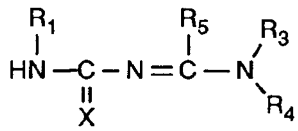

(V),

worin $R_1$, $R_3$, $R_4$, $R_5$ und X die für die Formel I angegebenen Bedeutungen haben, umsetzt und jeweils die entstehende Verbindung der Formel I isoliert oder in ein Säureadditionssalz überführt und dieses isoliert.

2. Verfahren gemäss Anspruch 1 zur Herstellung einer Verbindung der Formel I in freier Form.

3. Verfahren gemäss Anspruch 2 zur Herstellung einer Verbindung der Formel I, worin der Rest A ungesättigt und über ein Kohlenstoff-Ringglied an das den Rest $R_2$ tragende Kohlenstoffatom der Formel I gebunden ist.

4. Verfahren gemäss Anspruch 3 zur Herstellung einer Verbindung der Formel I, worin der Rest A aromatischen Charakter aufweist.

5. Verfahren gemäss Anspruch 4 zur Herstellung einer Verbindung der Formel I, worin der Rest A mindestens ein Stickstoffatom enthält.

6. Verfahren gemäss Anspruch 4 zur Herstellung einer Verbindung der Formel I, worin der Rest A ein bis drei Heteroatome aus der Gruppe, bestehend aus Sauerstoff, Schwefel und Stickstoff, enthält, wobei jeweils höchstens eines der Heteroatome ein Sauerstoff- oder ein Schwefelatom ist.

7. Verfahren gemäss Anspruch 5 zur Herstellung einer Verbindung der Formel I, worin der Rest A ein bis drei Heteroatome aus der Gruppe, bestehend aus Sauerstoff, Schwefel und Stickstoff, enthält, wobei stets mindestens ein Stickstoffatom in A enthalten ist und höchstens eines der gegebenenfalls weiteren in A enthaltenen Heteroatome ein Sauerstoffatom oder ein Schwefelatom ist.

8. Verfahren gemäss Anspruch 4 zur Herstellung einer Verbindung der Formel I, worin der Rest A ausgewählt ist aus der Gruppe, bestehend aus den Grundkörpern

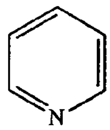 , 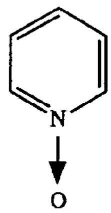 , 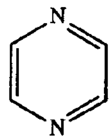 , 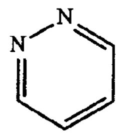 , 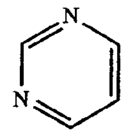 , 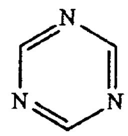 , 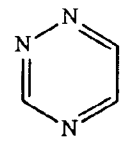 ,

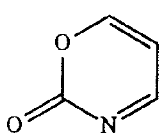 , 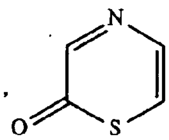 , 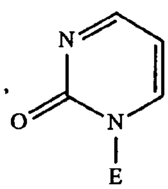 , 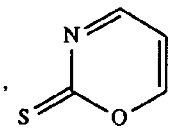 , 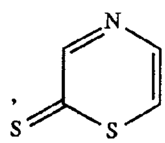 , 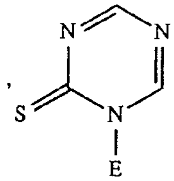 ,

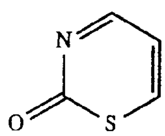 , 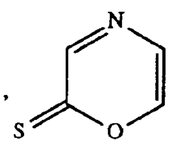 , 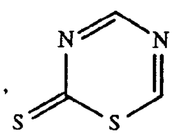 , , 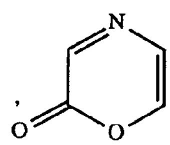 ,

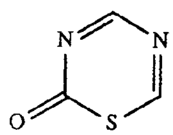 , 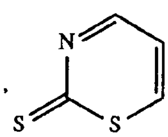 , 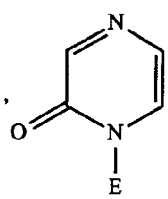 , 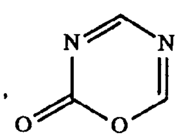 , 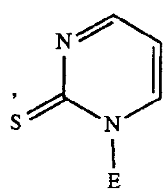 , 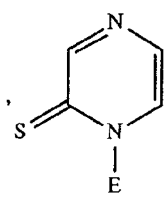 ,

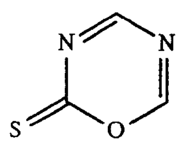 , 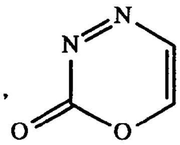 , 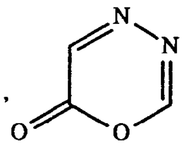 , 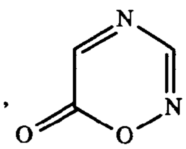 , 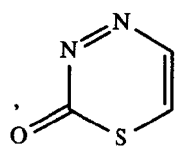 ,

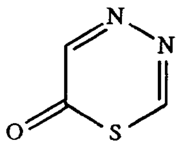 , 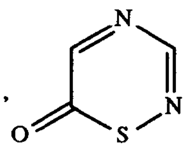 , 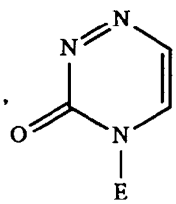 , 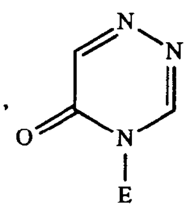 , 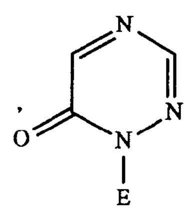 ,

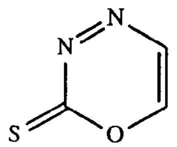 , 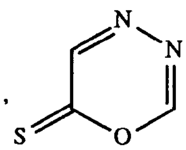 , 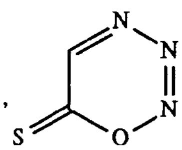 , 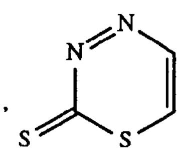 , 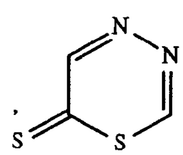 ,

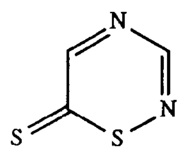 , 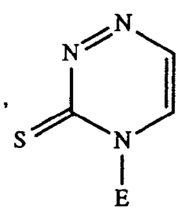 , 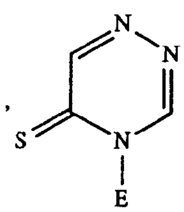 , 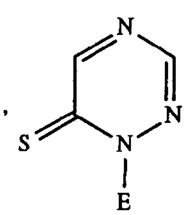 , 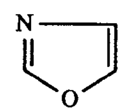 ,

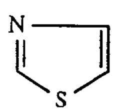 , 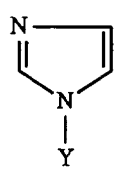 , 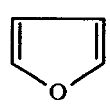 , 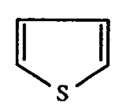 ,

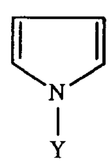 , 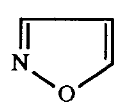 , 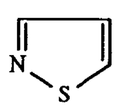 , 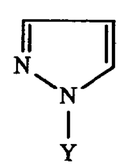 ,

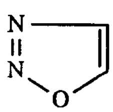 , 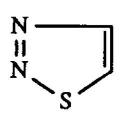 , 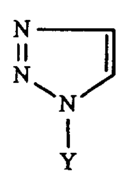 , 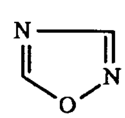 , 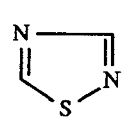 ,

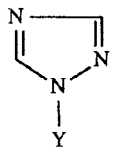 , 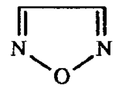 , 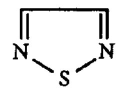 , 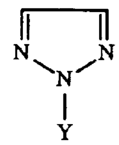 , 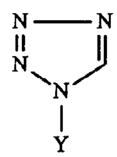 ,

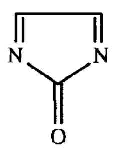 , 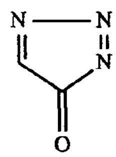 , 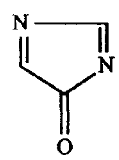 , 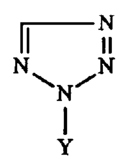 , 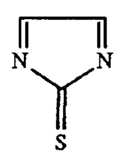 ,

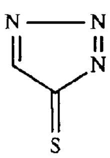 , 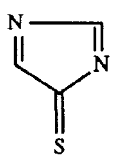 , 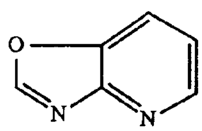 , 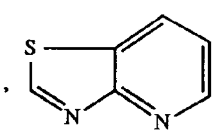 , 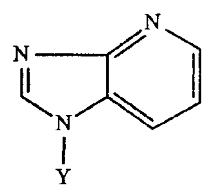 ,

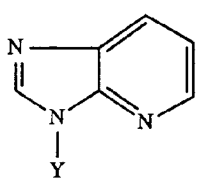 , 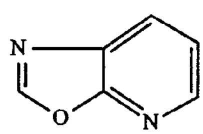 , 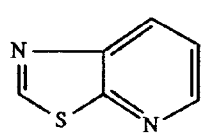 , 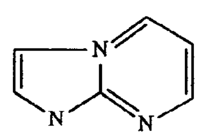 ,

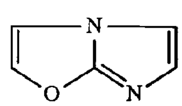 , 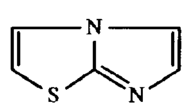 , 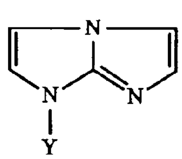 ,

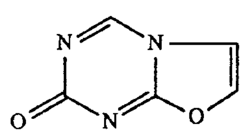 , 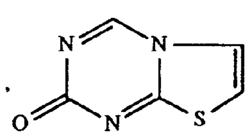 , 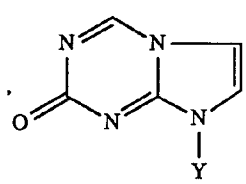 , 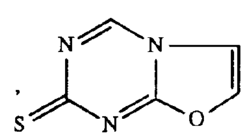 ,

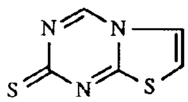 und 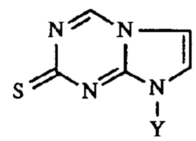 ,

welche unsubstituiert sind oder je nach den Substitutionsmöglichkeiten des Ringsystems bis zu vier der in Anspruch 1 definierten Substituenten tragen, worin E für $C_1$-$C_3$-Alkyl und Y für Wasserstoff, $C_1$-$C_3$-Alkyl oder Cyclopropyl stehen und welche über ein Kohlenstoffatom des Heterocyclus an das den Rest $R_2$ tragende Kohlenstoffatom der Formel I gebunden sind.

9. Verfahren gemäss Anspruch 8 zur Herstellung einer Verbindung der Formel I, worin der Rest A unsubstituiert ist oder einen oder zwei Substituenten, ausgewählt aus der Gruppe, bestehend aus Halogen, $C_1$-$C_3$-Alkyl, $C_1$-$C_3$-Halogenalkyl, $C_1$-$C_3$-Halogenalkoxy und $C_1$-$C_3$-Alkoxy, trägt.

**10.** Verfahren gemäss Anspruch 9 zur Herstellung einer Verbindung der Formel I, worin der Rest A ein von Pyridin oder Thiazol abgeleiteter Rest ist.

**11.** Verfahren gemäss Anspruch 10 zur Herstellung einer Verbindung der Formel I, worin der Rest A und-3-yl, 2-Halogenpyrid-5-yl, 2,3-Dihalogenpyrid-5-yl, 2-Halogen-1-oxido-5-pyridinio oder 2-Halogenthiazol-5-yl ist.

**12.** Verfahren gemäss Anspruch 11 zur Herstellung einer Verbindung der Formel I, worin =X für $=N-NO_2$, =N-CN oder $=CH-NO_2$ steht.

**13.** Verfahren gemäss Anspruch 12 zur Herstellung einer Verbindung der Formel I, worin
$R_1$ für Wasserstoff, $C_1$-$C_3$-Alkyl oder Cyclopropyl,
$R_2$ für Wasserstoff oder Methyl,
$R_3$ für $C_1$-$C_3$-Alkyl und
$R_4$ für $C_1$-$C_3$-Alkyl oder Ethoxycarbonylethyl oder
$R_3$ und $R_4$ gemeinsam für $-(CH_2)_4-$ oder $-(CH_2)_5-$ und
$R_5$ für Wasserstoff, Methyl, Ethyl, Phenyl $-CH^2OCH_3$, -CN, $-COOC_2H_5$ oder

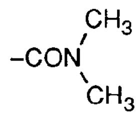

stehen.

**14.** Verfahren gemäss Anspruch 13 zur Herstellung einer Verbindung der Formel I, worin
$R_1$ für Methyl, Ethyl oder Cyclopropyl,
$R_2$ für Wasserstoff,
$R_3$ für Methyl,
$R_4$ für Methyl und
$R_5$ für Wasserstoff oder Methyl stehen.

**15.** Verfahren gemäss Anspruch 14 zur Herstellung einer Verbindung der Formel I, worin der Rest A 2-Chlorpyrid-5-yl ist.

**16.** Verfahren gemäss Anspruch 15 zur Herstellung einer Verbindung der Formel I, worin =X für $=N-NO_2$ oder $=CH-NO_2$ steht.

**17.** Verfahren gemäss Anspruch 1 zur Herstellung von N-(2-Chlorpyrid-5-ylmethyl)-N-methyl-N′-(N,N-dimethylaminomethylen)-N″-nitro-guanidin.

**18.** Verfahren gemäss Anspruch 1 zur Herstellung von 1-[N-(2-Chlorpyrid-5-ylmethyl)-N-methyl-amino]-1-(N,N-dimethylaminomethylenamino)-2-nitro-äthen.

**19.** Verfahren gemäss Anspruch 1 zur Herstellung von N-(2-Chlorpyrid-5-ylmethyl)-N-cyclopropyl-N′-(N,N-dimethylaminomethylen)-N″-nitro-guanidin.

**20.** Verfahren gemäss Anspruch 1 zur Herstellung einer Verbindung, ausgewählt aus der Gruppe, bestehend aus den Verbindungen
(b) N-(2-Chlorpyrid-5-ylmethyl)-N-methyl-N′-[1-(N,N-dimethylamino)ethyliden]-N″-nitro-guanidin,
(c) N-Pyrid-3-ylmethyl-N-methyl-N′-(N,N-dimethylaminomethylen)-N″-nitro-guanidin,
(d) N-(2-Chlorpyrid-5-ylmethyl)-N-methyl-N′-(N,N-dimethylaminomethylen)-N″-cyano-guanidin,
(f) 1-[N-(2-Chlorpyrid-5-ylmethyl)-N-ethyl-amino]-1-(N,N-dimethylaminomethylenamino)-2-nitro-äthen,
(g) 1-[N-(2-Chlorpyrid-5-ylmethyl)-N-cyclopropyl-amino]-1-(N,N-dimethylaminomethylenamino)-2-nitro-äthen,
(h) 1-[N-(2-Chlorthiazol-5-ylmethyl)-N-methyl-amino]-1-(N,N-dimethylaminomethylenamino)-2-nitro-äthen,
(i) 1-[N-(2-Chlorthiazol-5-ylmethyl)-N-ethyl-amino]-1-(N,N-dimethylaminomethylenamino)-2-nitro-äthen,
(j) 1-[N-(2-Chlorthiazol-5-ylmethyl)-N-cyclopropyl-amino]-1-(N,N-dimethylaminomethylenamino)-2-nitro-äthen,

(k) 1-[N-(2-Chlor-1-oxido-5-pydiniomethyl)-N-methyl-amino]-1-(N,N-dimethylaminomethylenamino)-2-nitro-äthen,
(l) 1-[N-(2-Chlor-1-oxido-5-pyridiniomethyl)-N-ethyl-amino]-1-(N,N-dimethylaminomethylenamino)-2-nitro-äthen,
(m) 1-[N-(2-Chlor-1-oxido-5-pyridiniomethyl)-N-cyclopropyl-amino]-1-(N,N-dimethylaminomethylenamino)-2-nitro-äthen,
(n) N-(2-Chlorthiazol-5-ylmethyl)-N-methyl-N′-(N,N-dimethylaminomethylen)-N″-nitro-guanidin,
(o) N-(2-Chlorthiazol-5-ylmethyl)-N-ethyl-N′-(N,N-dimethylaminomethylen)-N″-nitro-guanidin,
(p) N-(2-Chlorthiazol-5-ylmethyl)-N-cyclopropyl-N′-(N,N-dimethylaminomethylen)-N″-nitro-guanidin und
(q) N-(2-Chlorpyrid-5-ylmethyl)-N-ethyl-N′-(N,N-dimethylaminomethylen)-N″-nitro-guanidin.

**21.** Verfahren gemäss einem der Ansprüche 1 bis 20, dadurch gekennzeichnet, dass man die Umsetzung gemäss der Verfahrensvariante a) in einem inerten aprotischen Lösungsmittel, wie einem Kohlenwasserstoff, einem chlorierten Kohlenwasserstoff, einem Aether, einem Nitril oder einem Amid, bei 20 bis 150°C und unter atmosphärischem Druck ausführt.

**22.** Verfahren gemäss Anspruch 21, dadurch gekennzeichnet, dass man die Umsetzung bei 50-120°C in Tetrahydrofuran, Dioxan oder Toluol als Lösungsmittel durchführt.

**23.** Verfahren gemäss einem der Ansprüche 1 bis 20, dadurch gekennzeichnet, dass man die Umsetzung gemäss der Verfahrensvariante b) in Gegenwart von Natriumhydrid, Natriumamid, Kaliumcarbonat, Triäthylamin oder Diäthylanilin als Säureakzeptor und in N,N-Dimethylformamid, 1-Methylpyrrolidon, Dimethylsulfoxid, Sulfolan, Tetrahydrofuran oder Acetonitril als Lösungsmittel durchführt.

**24.** Verfahren gemäss Anspruch 23, dadurch gekennzeichnet, dass man die Umsetzung bei - 10 bis +30°C mit Natriumhydrid oder Natriumamid als Säureakzptor durchführt.

**25.** Schädlingsbekämpfungsmittel, dadurch gekennzeichnet, dass es mindestens eine Verbindung, erhältlich gemäss Anspruch 1, der Formel I, in freier Form oder in agrochemisch verwendbarer Säureadditionssalzform, als Wirkstoff und mindestens einen Hilfsstoff enthält.

**26.** Mittel gemäss Anspruch 25 zur Bekämpfung von Insekten und/oder Spinnentieren.

**27.** Verfahren zur Herstellung eines Mittels gemäss Anspruch 25 oder 26, dadurch gekennzeichnet, dass man den Wirkstoff mit dem (den) Hilfsstoff(en) innig vermischt.

**28.** Verwendung einer Verbindung, erhältlich gemäss Anspruch 1, der Formel I, in freier Form oder in agrochemisch verwendbarer Säureadditionssalzform, oder eines Mittels gemäss Anspruch 25 zur Bekämpfung von Schädlingen.

**29.** Verwendung gemäss Anspruch 28 zur Bekämpfung von Insekten und/oder Spinnentieren.

**30.** Verwendung gemäss Anspruch 28 zur Behandlung von Saatgut.

**31.** Verwendung einer Verbindung, erhältlich gemäss Anspruch 1, der Formel I, in freier Form oder in agrochemisch verwendbarer Säureadditionssalzform, zur Herstellung eines Mittels gemäss Anspruch 25 oder 26.

**32.** Verfahren zur Bekämpfung von Schädlingen, dadurch gekennzeichnet, dass man eine Verbindung, erhältlich gemäss Anspruch 1, der Formel I, in freier Form oder in agrochemisch verwendbarer Säureadditionssalzform, oder ein Mittel gemäss Anspruch 25 oder 26 auf die Schädlinge und/oder ihren Lebensraum appliziert.

**33.** Verfahren gemäss Anspruch 32 zur Bekämpfung von Insekten und/oder Spinnentieren.

**34.** Verfahren gemäss Anspruch 32 zum Schutz von Saatgut, dadurch gekennzeichnet, dass man das Saatgut oder die Saatfurche behandelt.

**35.** Saatgut, behandelt gemäss dem in Anspruch 34 beschriebenen Verfahren.

**36.** Verfahren zur Herstellung einer Verbindung der Formel

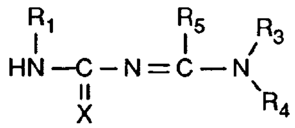

(V),

worin $R_1$, $R_3$, $R_4$, $R_5$ und X die in Anspruch I für die Formel I angegebenen Bedeutungen haben, dadurch gekennzeichnet, dass man eine Verbindung der Formel

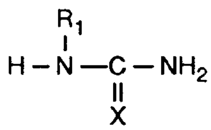

(IIa),

worin $R_1$ und X die für die Formel I angegebenen Bedeutungen haben, in einem inerten aprotischen Lösungsmittel mit einer Verbindung der Formel

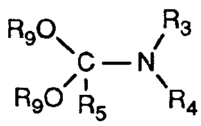

(III),

worin $R_3$, $R_4$ und $R_5$ die für die Formel I angegebenen Bedeutungen haben und $R_9$ $C_1$-$C_4$-Alkyl ist, umsetzt und die entstehende Verbindung der Formel V isoliert.

**37.** Verfahren gemäss Anspruch 36 zur Herstellung einer Verbindung, ausgewählt aus der Gruppe, bestehend aus den Verbindungen
N-Cyclopropyl-N′-(N,N-dimethylaminomethylen)-N″-nitro-guanidin,
N-(N,N-Dimethylaminomethylen)-N′-methyl-N″-nitro-guanidin und
1-(N,N-Dimethylaminomethylenamino)-1-(N-methylamino)-2-nitro-aethen.

Europäisches Patentamt

# EUROPÄISCHER RECHERCHENBERICHT

Nummer der Anmeldung

EP 92 81 0225

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D<br><br>D | EP-A-0 375 907 (NIHON TOKUSHU NOYAKU SEIZO K.K.)<br>* Ansprüche 1,7-11; Beispiele; Tabellen 1,2 *<br>& EP-A-0 383 091<br>--- | 1-37 | C07D213/40<br>C07D277/32<br>C07D213/61<br>C07D213/89<br>A01N43/40<br>A01N43/78<br>A01N47/42 |
| A,D | EP-A-0 376 279 (TAKEDA CHEMICAL INDUSTRIES, LTD. )<br>* Ansprüche 1,11,23-28; Beispiele; Tabelle 3 *<br>--- | 1-37 | |
| A | EP-A-0 254 859 (NIHON TOKUSHU NOYAKU SEIZO K.K.)<br>*Verbindung 16*<br>* Ansprüche 1,6-9 *<br>--- | 1-37 | |
| A | EP-A-0 418 199 (CIBA-GEIGY AG)<br>* das ganze Dokument *<br>----- | 1-37 | RECHERCHIERTE SACHGEBIETE (Int. Cl.5 )<br>C07D |

Der vorliegende Recherchenbericht wurde für alle Patentansprüche erstellt

| Recherchenort | Abschlußdatum der Recherche | Prüfer |
|---|---|---|
| DEN HAAG | 15 JULI 1992 | P. BOSMA |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)